# EUROPEAN PATENT APPLICATION

(11) **EP 3 882 884 A1**
(43) Date of publication of application: **22.09.2021**
(21) Application number: 19885826.8
(22) Date of filing: 30.10.2019
(51) Int. Cl.: G08G 1/16, B60W 40/08, B60W 50/12, A61B 5/00

(54) **INFORMATION PROCESSING DEVICE, INFORMATION PROCESSING METHOD, AND PROGRAM**

(30) Priority: 13.11.2018 JP 2018212727
(71) Applicant: Sony Group Corporation, Minato-ku, Tokyo 108-0075 (JP)
(72) Inventor: TAMORI Masahiro, Tokyo 108-0075 (JP); ARIKADO Tomohiro, Tokyo 108-0075 (JP); KOEDA Tatsuya, Tokyo 108-0075 (JP); OGAWA Yasufumi, Tokyo 108-0075 (JP)
(74) Representative: MFG Patentanwälte Meyer-Wildhagen Meggle-Freund Gerhard PartG mbB
(86) International application number: PCT/JP2019/042491
(87) International publication number: WO 2020/100584

(57) **Abstract**

The present disclosure relates to an Information processing apparatus, an information processing method, and a program for detecting a driver's abnormal physical condition during driving to implement processing in consideration of safety.

Vital data of the driver of a vehicle is acquired and the vehicle is controlled to a safe state according to an automatic driving level in a case where a determination result that an abnormal physical condition is occurring in the driver is acquired on the basis of the vital data. The present disclosure can be applied to an in-vehicle system.

## Description

### TECHNICAL FIELD

The present disclosure relates to an information processing apparatus, an information processing method, and a program, and in particular relates to an information processing apparatus, an information processing method, and a program for detecting a driver's abnormal physical condition during driving to implement processing in consideration of safety.

### BACKGROUND ART

A technology of transmitting vital data that is biometric information of a driver who drives a vehicle to a help net center, and monitoring a physical condition of the driver in the help net center has been proposed (see Patent Document 1).

### CITATION LIST

### PATENT DOCUMENT

Patent Document 1: Japanese Patent Application Laid-Open No. 2013-171569

### SUMMARY OF THE INVENTION

### PROBLEMS TO BE SOLVED BY THE INVENTION

However, in the technology of Patent Document 1, the biometric information is read as the driver holds a fingertip against a sensor. The vital data is not detected unless the driver consciously holds the fingertip against the sensor and is not transmitted to the help net center.

For this reason, when the driver suddenly loses consciousness while driving, the vital data (biometric information) is not transmitted to the help net center, so proper driving control in consideration of safety cannot be implemented even if an abnormal physical condition such as loss of consciousness occurs.

The present disclosure has been made in view of such a situation and is in particular intended to acquire vital data and implement proper driving control in consideration of safety when an abnormal physical condition occurs.

### SOLUTIONS TO PROBLEMS

An information processing apparatus according to one aspect of the present disclosure is an information processing apparatus including a vital data acquisition unit configured to acquire vital data of a driver of a vehicle, and an abnormal situation processing unit configured to control the vehicle to a safe state according to an automatic driving level in a case of acquiring a determination result that an abnormal physical condition is occurring in the driver on the basis of the vital data.

An information processing method and a program according to one aspect of the present disclosure correspond to the information processing apparatus.

In one aspect of the present disclosure, vital data of the driver of a vehicle is acquired and the vehicle is controlled to a safe state according to an automatic driving level in a case where a determination result that an abnormal physical condition is occurring in the driver is acquired on the basis of the vital data.

### BRIEF DESCRIPTION OF DRAWINGS

Fig. 1 is a diagram illustrating a configuration for detecting a line-of-sight.
Fig. 2 is a diagram illustrating an example in which a driver is transported to a medical institution when an abnormal physical condition occurs in the driver.
Fig. 3 is a diagram illustrating an example in which a vehicle is stopped at a safe place when an abnormal physical condition occurs in the driver.
Fig. 4 is a block diagram illustrating a configuration example of a transportation system of the present disclosure.
Fig. 5 is a block diagram illustrating a configuration example of a vehicle control system of the present disclosure.
Fig. 6 is a block diagram illustrating a configuration example of a report recipient terminal of the present disclosure.
Fig. 7 is a block diagram illustrating a configuration example of a medical operator terminal of the present disclosure.
Fig. 8 is a block diagram illustrating a configuration example excerpting a configuration for implementing safe driving control in Fig. 5.
Fig. 9 is a flowchart illustrating automatic driving control processing.
Fig. 10 is a flowchart illustrating abnormal driving person determination processing.
Fig. 11 is a flowchart illustrating abnormal situation processing.
Fig. 12 is a flowchart illustrating automatic driving level 5 corresponding processing.
Fig. 13 is a flowchart illustrating automatic driving level 4 corresponding processing.
Fig. 14 is a flowchart illustrating automatic driving level 3 corresponding processing.
Fig. 15 is a flowchart illustrating emergency stop support processing.
Fig. 16 is a diagram illustrating a configuration example of a general-purpose computer.

### MODE FOR CARRYING OUT THE INVENTION

A favorable embodiment of the present disclosure will be described in detail with reference to the appended drawings. Note that, in the present specification and drawings, redundant description of configuration elements having substantially the same functional configuration is omitted by providing the same sign.

Hereinafter, modes for carrying out the present technology will be described. Description will be given in the following order.
1. Outline of Present Disclosure
2. Configuration Example of Transportation System of Present Disclosure
3. Configuration Example of Vehicle Control System That Controls Vehicle of Present Disclosure
4. Configuration Example of Report Recipient Terminal of Present Disclosure
5. Configuration Example of Medical Operator Terminal of Present Disclosure
6. Configuration Example of Automatic Driving Control Unit That Detects Abnormal Physical Condition on Basis of Vital Data and Implements Driving Control in Consideration of Safety
7. Automatic Driving Control Processing
8. Driver Abnormality Determination Processing
9. Abnormal Situation Processing
10. Automatic Driving Level 5 Corresponding Processing
11. Automatic Driving Level 4 Corresponding Processing
12. Automatic Driving Level 3 Corresponding Processing
13. Emergency Stop Support Processing
14. Example of Execution by Software

### <<1. Outline of Present Disclosure>>

### <Detection of Vital Data>

An outline of the present disclosure will be described.

A vehicle of the present disclosure detects vital data of a driver and performs driving control in consideration of safety of the driver and passengers, vehicles around the vehicle, passersby, and the like on the basis of whether or not an abnormal physical condition is occurring in the driver.

Examples of the vital data of the driver include presence/absence of line-of-sight detection, heartbeat, pulse, body surface pulse wave, and body temperature.

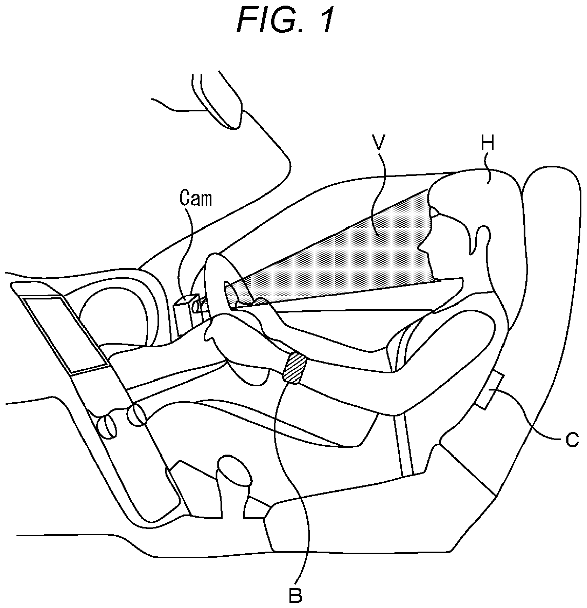

The presence or absence of line-of-sight detection is determined, for example, by capturing a range V near the face of a driver H using a camera Cam provided in an upper part of a steering and at a position facing the driver H, and determining whether or not it is a state in which a pupil detected on the basis of the position of a pupil of the driver in the captured image is not open, and is a state in which the line-of-sight continues with respect to a predetermined direction for a predetermined time, as illustrated in Fig. 1. Note that, in a case where a line-of-sight direction does not change for the predetermined time or more, it is expected that the driver loses consciousness. Therefore, the presence or absence of the line-of-sight detection is determined according to whether or not the driver is staring for an appropriate time after considering a state of the detected pupil of the eye and the like.

The camera Cam desirably includes not only a so-called image sensor but also a time of flight (ToF) sensor.

More specifically, in the case of an image obtained by the image sensor, there is a possibility that the entire face is exposed to strong light by headlights of an oncoming vehicle at night, for example, and the image of the entire face is overexposed and each organ forming the face may not be able to be recognized.

In contrast, in the case of a distance image obtained by the ToF sensor, information of a distance from the camera Cam to each position on a surface of the face is obtained, and unevenness of the surface of the face can be recognized. Therefore, each organ forming the face can be recognized on the basis of information of the unevenness of the surface of the face.

For this reason, it is desirable that the camera Cam has the configuration provided with the image sensor and the ToF sensor, and uses the image by the image sensor and the distance image by the ToF sensor depending on a situation. Furthermore, by combining the image sensor and the ToF sensor, robustness may be improved or a processing time may be shortened.

Here, for the heartbeat, pulse, and body temperature, the heartbeat, pulse, and body temperature may be directly detected by a wearable device B that the driver H in Fig. 1 wears on its arm, for example. Furthermore, the body surface pulse wave may be detected by a sensor C that is attached to a seat and comes in contact with a back of the driver.

Moreover, the heartbeat and pulse may be measured in a noncontact manner with the driver, using, for example, a microwave sensor. The microwave sensor is a sensor that uses characteristic that a surface of a human body minutely vibrates due to movement of heart and lungs. For example, the driver is irradiated with a microwave of 24.125 GHz, a reflected microwave is measured, and a heartbeat (pulse) is detected using the Doppler effect caused by the minute vibration of the human body surface from a measurement result.

Furthermore, the body temperature may be detected by a warmth sensor.

Note that the vital data may be biometric information other than the line-of-sight detection, heartbeat, pulse, body surface pulse wave, and body temperature as long as the biometric information is data by which the physical condition of the driver is confirmable.

### <Driving Control According to Automatic Driving Level>

The vehicle of the present disclosure is premised on being driven by automatic driving, and driving control is performed according to an automatic driving level.

Here, the automatic driving level classifies a level of the automatic driving, and is classified into, for example, levels 0 to 5.

Level 0 is an automatic driving level in which the driver performs all of driving operations, and is not substantially the automatic driving.

Level 1 is an automatic driving level in which either a driving operation related to acceleration or deceleration or a driving operation related to a steering operation among the driving operations is controlled.

Level 2 is an automatic driving level in which the driving operation related to acceleration or deceleration and the driving operation related to a steering operation among the driving operations are controlled in cooperation.

Level 3 is an automatic driving level in which all the driving operations are controlled in a specific place such as an expressway. Note that, at the level 3, it is premised that the driver performs the driving operation in an emergency.

Level 4 is an automatic driving level in which all the driving operations are controlled including in an emergency in a specific place such as an expressway.

Level 5 is an automatic driving level in which all the driving operations are controlled in all situations, and is a level that is so-called fully automatic driving.

As described above, the technology for implementing the automatic driving levels of the levels 0 to 2 is generally called driving assistance technology because it is the technology in which the driver mainly performs the driving operations.

In contrast, the technology for implementing the automatic driving levels of the levels 3 to 5 is generally called automatic driving technology because it is the technology in which the driver becomes free from mainly performing the driving operations in a specific place.

The driving control of the present disclosure determines the presence or absence of an abnormal physical condition on the basis of the vital data of the driver, and implements the driving control in consideration of safety according to the automatic driving level.

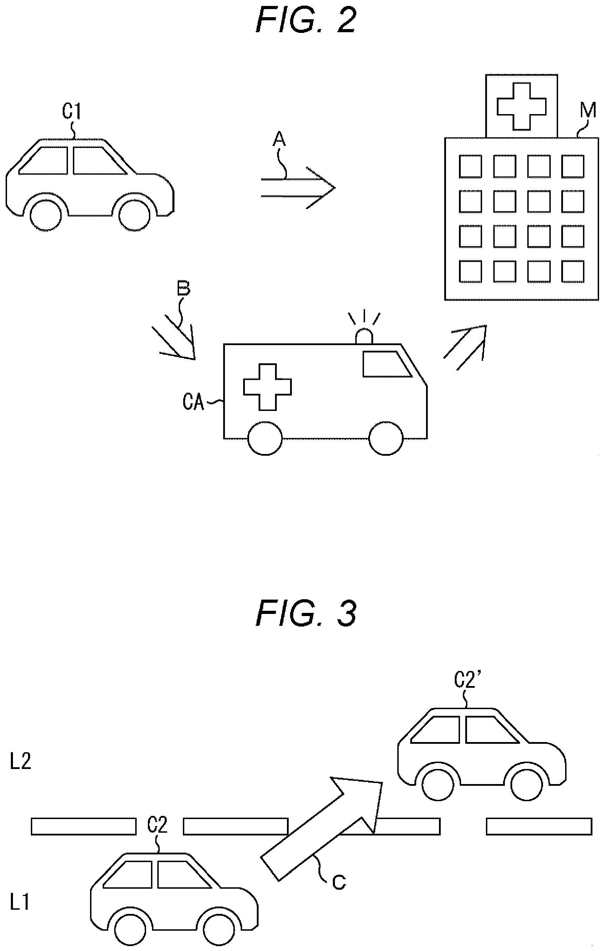

More specifically, for example, in a case where it is detected that an abnormal physical condition is occurring in the driver on the basis of the vital data in a state where a vehicle C1 is traveling as in Fig. 2, the driving is the fully automatic driving at the automatic driving level 5. Therefore, the vehicle C1 transports the driver to a medical institution M, as illustrated by the arrow A.

Furthermore, in a case where the driver is in a serious physical condition and requires a rescue by an emergency vehicle, and in the case of the automatic driving level 5, the vehicle C1 transports the driver to a merging point with an emergency vehicle CA, as illustrated by the arrow B. Then, the emergency vehicle CA (emergency personnel, a doctor, or the like) treats the driver of the vehicle C1 as necessary and transports the driver to the medical institution M.

Moreover, in the case of the automatic driving level 4, the automatic driving is possible, and replacement of the driver is not necessary, the vehicle C1 transports the driver to the medical institution M, similarly to the case of the automatic driving level 5.

Moreover, in the case of the automatic driving level 4, replacement of the driver is necessary but when there is no replacement, for example, a vehicle C2 traveling in a lane L1 as illustrated on the left in Fig. 3 is stopped at a safe place on a shoulder L2 as illustrated by a vehicle C2' on the right in Fig. 3. Then, an emergency vehicle is arranged to this stop position, and the emergency vehicle CA goes to the stop position and transports the driver to the medical institution M after the emergency vehicle CA (emergency personnel, a doctor, or the like) treats the driver in the abnormal physical condition.

Here, in the case of the automatic driving level 4, in an environment where the automatic driving is not possible, and replacement of the driver is necessary and the replacement is possible, a replaced driver drives the vehicle C2' and transports the driver in the abnormal physical condition to the medical institution M or the merging point with the emergency vehicle CA.

Furthermore, in the case of the automatic driving level 3, the automatic driving cannot be continued in the situation where the driver cannot drive the vehicle. Therefore, occurrence of the abnormal physical condition of the driver is detected, the vehicle C2 traveling in the lane L1 as illustrated in Fig. 2 is stopped at a safe place of the shoulder L2 and wait for transportation by the emergency vehicle CA, as illustrated by the vehicle C2'.

Here, in the environment where the automatic driving is not possible, and replacement of the driver is necessary and the replacement is possible, a replaced driver drives a vehicle C12' and transports the driver in the abnormal physical condition to the medical institution M or the merging point with the emergency vehicle CA, as in the case of the automatic driving level 4.

Moreover, in the case of the automatic driving levels 0 to 2, when the abnormal physical condition in the driver is detected, a safe place cannot be specified, but the hazard lights are turned on and the vehicle is stopped on a driving lane and waits for transportation by the emergency vehicle CA.

Here, in a case where replacement of the driver is possible, a replaced driver drives the vehicle and transports the driver to the medical institution M or the merging point with the emergency vehicle CA, as in the case of the automatic driving level 3 or 4.

In this way, the vital data of the driver is acquired and the presence or absence of the abnormal physical condition is detected, and in a case where the abnormal physical condition is detected, processing considering safety can be implemented by the driving control according to the automatic driving level.

### <<2. Configuration Example of Transportation System of Present Disclosure>>

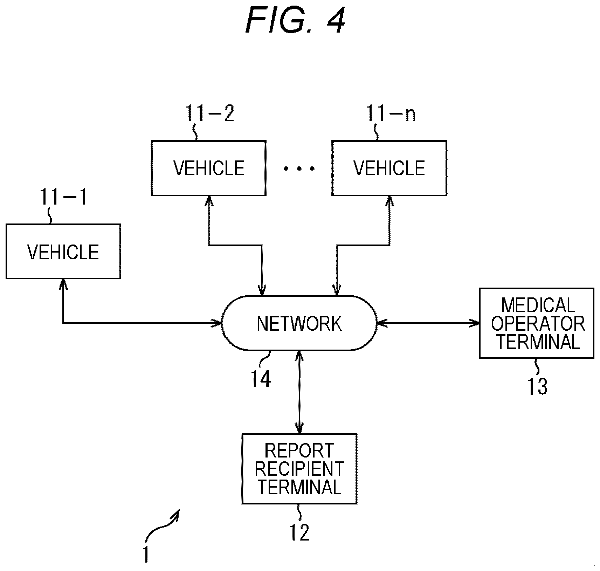

Next, a configuration example of a transportation system that acquires the vital data of the driver of the present disclosure and implements proper driving control in consideration of safety when an abnormal physical condition occurs will be described with reference to Fig. 4.

A transportation system 1 in Fig. 4 includes vehicles 11-1 to 11-n, a report recipient terminal 12, a medical operator terminal 13, and a network 14. The vehicles 11-1 to 11-n, the report recipient terminal 12, and the medical operator terminal 13 are configured to be able to communicate with one another via the network 14.

The vehicles 11-1 to 11-n are vehicles driven at various automatic driving levels of the automatic driving levels 0 to 5. Note that, in a case where it is not necessary to distinguish the vehicles 11-1 to 11-n, they are simply referred to as vehicle(s) 11, and other configurations are also similarly referred to.

When the driver gets into the vehicle 11, the vehicle 11 identifies the driver, and acquires the presence or absence of the line-of-sight detection and the vital data of the driver and determines the presence or absence of the abnormal physical condition. When the vehicle 11 determines that there is the abnormal physical condition on the basis of the vital data of the driver, the vehicle 11 notifies the report recipient terminal 12 of position information, the vital data, information that specifies the driver, and information that notifies the abnormal physical condition of the driver.

Note that the vital data includes all of the line-of-sight detection, heartbeat, pulse, body surface pulse wave, and body temperature of the driver. However, hereinafter, the line-of-sight detection will be described as independent data of the vital data. That is, hereinafter, in the case of referring to vital data, the vital data does not include line-of-sight detection result information but indicates the heartbeat, pulse, body surface pulse wave, and body temperature. Furthermore, the information that specifies the driver may be any information as long as the information can specify the individual driver. More specifically, the information that specifies the driver may be, for example, a face image of the driver, or biometric information such as a fingerprint or a retinal pattern of the driver, or registered personal information itself such as a name or an age of the driver when the information that specifies the driver is stored in association with the personal information on the basis of the biometric information.

The vehicle 11 acquires information of the medical institution (including position information) or position information of the merging point with the emergency vehicle transmitted from the medical operator terminal 13 via the report recipient terminal 12, and implements processing considering safety according to the automatic driving level, on the basis of the position information, the vital data, and the information that specifies the driver.

The report recipient terminal 12 is a terminal operated by a report recipient who receives a report of the abnormal physical condition of the driver, and receives the report of the abnormal physical condition of the driver and acquires the position information, the vital data, and the information that specifies the driver transmitted together with the report. The report recipient is, for example, an operator or the like in the transportation system.

The report recipient terminal 12 transmits the received position information, vital data, and information that specifies the driver to the medical operator terminal 13.

The report recipient terminal 12 acquires the position information of the medical institution capable of providing necessary treatment to the driver in the abnormal physical condition and information of necessity of an emergency vehicle, which are transmitted from the medical operator terminal 13, on the basis of the position information, the vital data, and the information that specifies the driver.

In a case where arrangement of the emergency vehicle is requested by the medical operator terminal 13, the report recipient terminal 12 specifies an appropriate merging point between the vehicle 11 and the emergency vehicle in consideration of a route for transporting the driver to the medical institution from the position information of the vehicle 11 driven by the driver in the abnormal physical condition and the position information of the specified medical institution. Then, the report recipient terminal 12 transmits information of the merging point with the emergency vehicle together with the information of the medical institution capable of providing necessary treatment to the driver in the abnormal physical condition to the vehicle 11, and arranges the emergency vehicle to rush to the merging point.

Furthermore, in a case where the arrangement of the emergency vehicle is not requested by the medical operator terminal 13, the report recipient terminal 12 transmits the position information of the medical institution capable of providing necessary treatment to the driver in the abnormal physical condition to the vehicle 11. Note that, in this case, when there is a request of the emergency vehicle from the vehicle 11, such as when replacement of the driver is necessary in the vehicle 11 but there is no replacement, the report recipient terminal 12 arranges the emergency vehicle to rush to the site where the vehicle 11 is stopped.

The medical operator terminal 13 is a terminal operated by the medical operator, and acquires the vital data and the information that specifies the driver transmitted from the report recipient terminal 12 and searches for a medical record of the driver in the abnormal physical condition on the basis of the information that specifies the driver.

The medical operator is, for example, medical personnel such as a doctor, a dentist, a pharmacist, or a nurse, and is a person who can judge medical treatment required for the driver in the abnormal physical condition from the vital data, the information of the medical record, and the like.

In a case where the medical operator terminal 13 can find the medical record of the driver, the medical operator terminal 13 presents the medical record together with the vital data to the medical operator and requests the medical operator to specify the treatment necessary for the driver in the abnormal physical condition and the medical institution capable of providing the treatment, and judge necessity of the emergency vehicle and the like.

In response to the request, the medical operator judges the treatment necessary for the driver in the abnormal physical condition, specifies the corresponding treatment and the medical institution capable of providing the treatment, and judges the necessity of the emergency vehicle.

Then, the medical operator operates and causes the medical operator terminal 13 to transmit the position information of the specified medical institution and the information of the necessity of the emergency vehicle to the report recipient terminal 12.

In a case where there is the information of the medical record of the driver in the abnormal physical condition, the medical institution that holds the medical record is likely to be the driver's regular hospital or the like. Therefore, if the treatment for the driver in the current abnormal physical condition is possible, the hospital may be preferentially specified as the medical institution on the destination even if the hospital is a little far away.

Furthermore, in a case where there is no medical record of the driver, the medical operator terminal 13 requests the medical operator to specify the necessary medical treatment and the medical institution capable of providing the treatment, and judge the necessity of the emergency vehicle on the basis of the vital data.

Moreover, the vehicle 11, the report recipient terminal 12, and the medical operator terminal 13 are configured to be able to call one another.

Therefore, in the series of processing, in a case where either the driver or the passenger of the vehicle 11 can make a call, a mutual call with the report recipient and the medical operator is implemented.

Therefore, in a case where a call is possible, the medical operator comprehensively judges necessary medical treatment on the basis of the information of the medical record (in a case where there is the information of the medical record of the driver), the vital data, and call content, and can specify the medical institution and judge the necessity of the emergency vehicle.

With such a communication function, more appropriate treatment can be judged for the driver in the abnormal physical condition.

Furthermore, the medical operator terminal 13 may have an agent function that proposes necessary treatment to the driver in the abnormal physical condition.

That is, the agent function of the medical operator terminal 13 may comprehensively determine the necessary medical treatment and the medical institution capable of providing the treatment, and propose the medical institution and the necessity of the emergency vehicle, on the basis of the information of the medical record (in a case where there is the information of the medical record of the driver), the vital data, and the call content (in a case where a call is possible).

With such a series of transportation system, the proper driving control in consideration of safety can be implemented even in the situation where the driver who drives the vehicle 11 falls in the abnormal physical condition during driving.

### <<3. Configuration Example of Vehicle Control System That Controls Vehicle of Present Disclosure>>

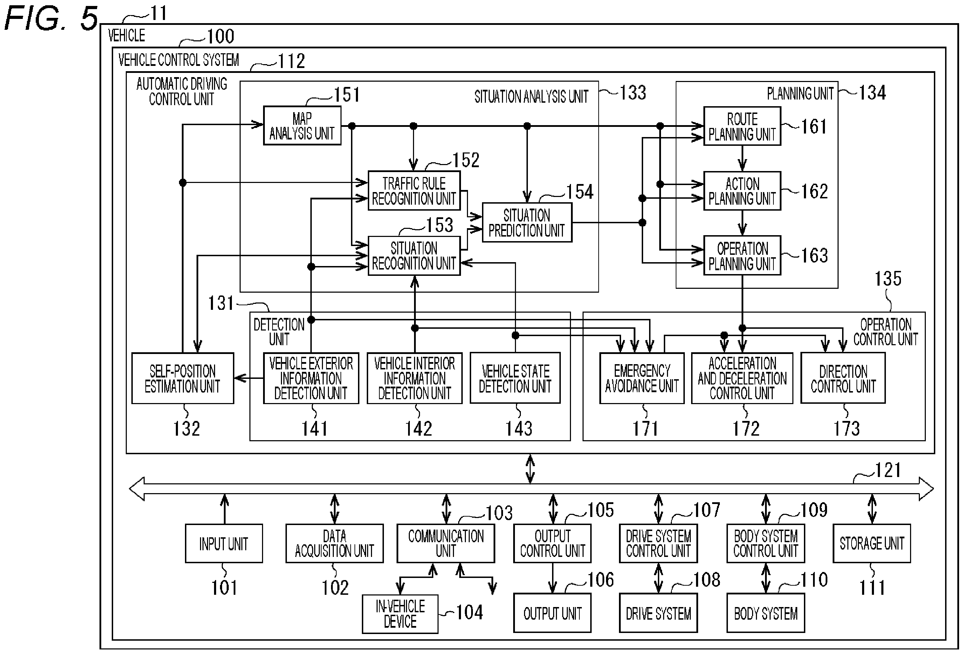

Next, a vehicle control system of a vehicle of the present disclosure will be described with reference to the block diagram in Fig. 5.

Fig. 5 is a block diagram illustrating a configuration example of schematic functions of a vehicle control system 100 of the vehicle 11 to which the present technology can be applied.

Note that, hereinafter, in a case of distinguishing the vehicle 11 provided with the vehicle control system 100 from other vehicles, the vehicle 11 will be referred to as user's car or user's vehicle.

The vehicle control system 100 includes an input unit 101, a data acquisition unit 102, a communication unit 103, an in-vehicle device 104, an output control unit 105, an output unit 106, a drive system control unit 107, a drive system 108, a body system control unit 109, a body system 110, a storage unit 111, and an automatic driving control unit 112. The input unit 101, the data acquisition unit 102, the communication unit 103, the output control unit 105, the drive system control unit 107, the body system control unit 109, the storage unit 111, and the automatic driving control unit 112 are connected to one another via a communication network 121. The communication network 121 includes, for example, an on-board communication network conforming to an arbitrary standard such as a controller area network (CAN), a local interconnect network (LIN), a local area network (LAN), or FlexRay (registered trademark), a bus, and the like. Note that the units of the vehicle control system 100 may be directly connected without the communication network 121.

Note that, hereinafter, the case where the units of the vehicle control system 100 perform communication via the communication network 121, the description of the communication network 121 is omitted. For example, the case where the input unit 101 and the automatic driving control unit 112 perform communication via the communication network 121 will be simply described as the input unit 101 and the automatic driving control unit 112 performing communication.

The input unit 101 includes a device used by a passenger to input various data, instructions, and the like. For example, the input unit 101 includes operation devices such as a touch panel, a button, a microphone, a switch, and a lever, an operation device capable of inputting data, instructions, and the like by a method other than a manual operation, such as voice or gesture, and the like. Furthermore, for example, the input unit 101 may be a remote control device using infrared rays or other radio waves, or an externally connected device such as a mobile device or a wearable device corresponding to the operation of the vehicle control system 100. The input unit 101 generates an input signal on the basis of the data, instructions, and the like input by the passenger, and supplies the input signal to each unit of the vehicle control system 100.

The data acquisition unit 102 includes various sensors and the like that acquire data to be used for the processing of the vehicle control system 100, and supplies the acquired data to each unit of the vehicle control system 100.

For example, the data acquisition unit 102 includes various sensors for detecting the state of the user's car and the like. Specifically, for example, the data acquisition unit 102 includes a gyro sensor, an acceleration sensor, an inertial measurement device (IMU), sensors for detecting an operation amount of an accelerator pedal, an operation amount of a brake pedal, a steering angle of a steering wheel, an engine speed, a motor speed, a rotation speed of wheels, or the like, and the like.

Furthermore, for example, the data acquisition unit 102 includes various sensors for detecting information outside the user's car. Specifically, for example, the data acquisition unit 102 includes imaging devices such as a time of flight (ToF) camera, a stereo camera, a monocular camera, an infrared camera, and other cameras. Furthermore, for example, the data acquisition unit 102 includes an environment sensor for detecting a weather, a meteorological phenomenon, or the like, and an ambient information detection sensor for detecting an object around the user's car. The environment sensor includes, for example, a raindrop sensor, a fog sensor, a sunshine sensor, a snow sensor, and the like. The ambient information detection sensor includes, for example, an ultrasonic sensor, a radar device, a light detection and ranging or laser imaging detection and ranging (LiDAR) device, a sonar, or the like.

Moreover, the data acquisition unit 102 includes, for example, various sensors for detecting a current position of the user's car. Specifically, the data acquisition unit 102 includes a global navigation satellite system (GNSS) receiver that receives a GNSS signal from a GNSS satellite, a real time kinematic (RTK)-global positioning system (GPS), a network RTK, or the like.

Furthermore, for example, the data acquisition unit 102 includes various sensors for detecting information inside the vehicle. Specifically, for example, the data acquisition unit 102 includes imaging devices (time of flight (ToF) camera, stereo camera, monocular camera, infrared camera, other cameras, and the like) that image the driver, a biometric sensor that detects the biometric information of the driver, a microphone that collects sound in the vehicle, and the like. The biometric sensor is provided, for example, on a seating surface, a steering wheel, or the like, and detects the biometric information of a passenger sitting on a seat or the driver holding the steering wheel.

The communication unit 103 communicates with the in-vehicle device 104 and various devices outside the vehicle, a server, a base station, and the like, transmits data supplied from each unit of the vehicle control system 100, and supplies received data to each unit of the vehicle control system 100. Note that a communication protocol supported by the communication unit 103 is not especially limited, and the communication unit 103 can support a plurality of types of communication protocols.

For example, the communication unit 103 performs wireless communication with the in-vehicle device 104, using a wireless LAN, Bluetooth (registered trademark), near field communication (NFC), a wireless USB (WUSB), or the like. Furthermore, for example, the communication unit 103 performs wired communication with the in-vehicle device 104, using a universal serial bus (USB), a high-definition multimedia interface (HDMI), a mobile high-definition link (MHL), or the like via a connection terminal (not illustrated) (and a cable if necessary).

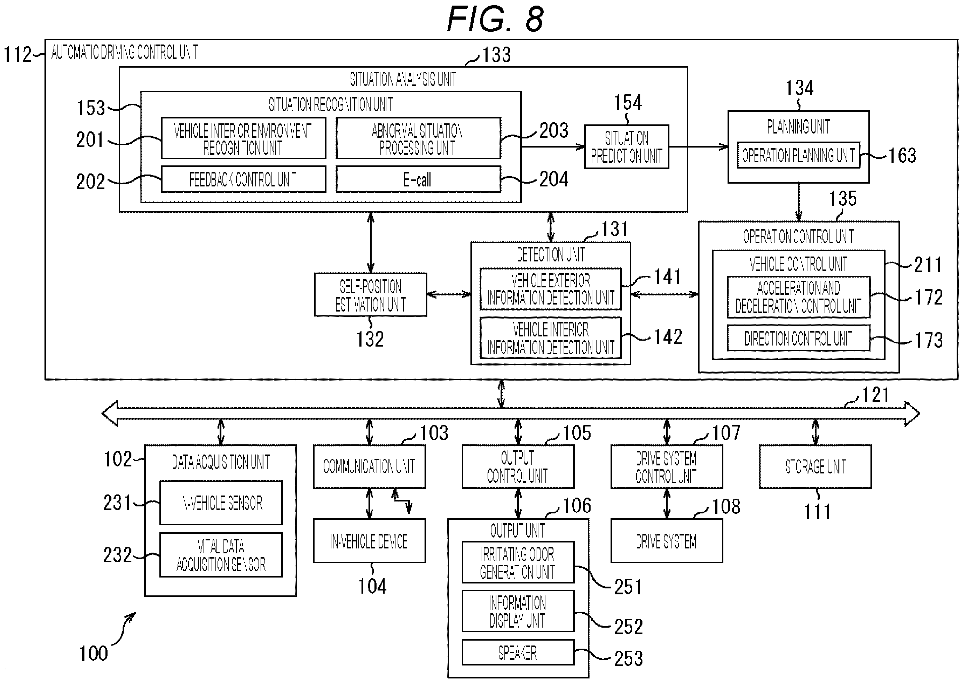

Moreover, for example, the communication unit 103 communicates with a device (for example, an application server or a control server) existing on an external network (for example, the Internet, a cloud network, or a company specific network) via a base station or an access point. Furthermore, for example, the communication unit 103 communicates with a terminal (for example, a terminal of a pedestrian or a shop, or a machine type communication (MTC) terminal) existing in the vicinity of the user's car, using a peer to peer (P2P) technology. Moreover, for example, the communication unit 103 performs V2X communication such as vehicle to vehicle communication, vehicle to infrastructure communication, vehicle to home communication, and vehicle to pedestrian communication. Furthermore, for example, the communication unit 103 includes a beacon reception unit, and receives a radio wave or an electromagnetic wave transmitted from a wireless station or the like installed on a road, and acquires information such as a current position, congestion, traffic regulation, or required time. Moreover, the communication unit 103 is controlled by an E-call 204 (Fig. 8) to be described below and transmits an occurrence position (GPS coordinates) where an accident has occurred to a center (for example, the report recipient terminal 12) located on an external network and which contacts with a police station, a hospital, or the like in conjunction with an operating state of a sensor that detects a collision of an airbag or the like.

The in-vehicle device 104 includes, for example, a mobile device or a wearable device of a passenger, an information device carried in or attached to the user's vehicle, a navigation device for searching for a route to an arbitrary destination, and the like.

The output control unit 105 controls output of various types of information to the passenger of the user's car or to the outside of the vehicle. The output control unit 105 controls output of visual information (for example, image data) and auditory information (for example, sound data) from the output unit 106 by generating an output signal including at least one of the visual information or the auditory information and supplying the output signal to the output unit 106, for example. Specifically, for example, the output control unit 105 synthesizes image data captured by different imaging devices of the data acquisition unit 102 to generate a bird's-eye view image, a panoramic image, or the like, and supplies an output signal including the generated image to the output unit 106. Furthermore, for example, the output control unit 105 generates sound data including a warning sound, a warning message, or the like for dangers of collision, contact, entry to a dangerous zone, or the like and supplies an output signal including the generated sound data to the output unit 106.

The output unit 106 includes a device capable of outputting the visual information or the auditory information to the passenger of the user's car or to the outside of the vehicle. For example, the output unit 106 includes a display device (including an information display unit 252 (Fig. 8)), an instrument panel, an audio speaker, headphones, a wearable device such as a glasses-type display worn by the passenger, a projector, a lamp, or the like. The display device included in the output unit 106 may be, for example, a head-up display (HUD), a transmission-type display, or a display for displaying the visual information in a field of view of the driver, such as a device having an augmented reality (AR) display function, in addition to a device having a normal display. Furthermore, the output unit 106 has a configuration having a feedback function that encourages arousal when the driver's line-of-sight cannot be detected, and inattentive driving or dozing driving is predicted, for example. Examples of the configuration having the feedback function include a device (information display unit 252 (Fig. 8) or the like) that displays visual information in a field of view of the driver, a speaker 253 (Fig. 8) that outputs sound, a mechanism that encourages arousal by a conversation of a dialog agent with the driver (for example, asking the name and date of birth to cause the driver to make a response, and determining whether or not the response is correct) using a microphone and a speaker or the like, a mechanism that vibrates a seat belt, a mechanism that vibrates the steering wheel, a mechanism that vibrates the seat, and an irritating odor generation unit 251 (Fig. 8) that generates an irritating odor.

The drive system control unit 107 controls the drive system 108 by generating various control signals and supplying the control signals to the drive system 108. Furthermore, the drive system control unit 107 supplies a control signal to each unit other than the drive system 108 to issue notification of a control state of the drive system 108, or the like, as needed.

The drive system 108 includes various devices related to the drive system of the user's car. For example, the drive system 108 includes a drive force generation device for generating a drive force of an internal combustion engine or a drive motor, a drive force transmission mechanism for transmitting the drive force to the wheels, a steering mechanism for adjusting the steering angle, a braking device for generating a braking force, an antilock brake system (ABS), an electronic stability control (ESC), an electric power steering device, and the like.

The body system control unit 109 controls the body system 110 by generating various control signals and supplying the control signals to the body system 110. Furthermore, the body system control unit 109 supplies a control signal to each unit other than the body system 110 and notifies a control state of the body system 110, or the like, as needed.

The body system 110 includes various body-system devices mounted on a vehicle body. For example, the body system 110 includes a keyless entry system, a smart key system, a power window device, a power seat, a steering wheel, an air conditioner, various lamps (for example, headlights, backlights, brake lights, blinkers, fog lights, and the like), and the like.

The storage unit 111 includes, for example, a magnetic storage device such as a read only memory (ROM), a random access memory (RAM), and a hard disc drive (HDD), a semiconductor storage device, an optical storage device, a magneto-optical storage device, and the like. The storage unit 111 stores various programs, data, and the like used by each unit of the vehicle control system 100. For example, the storage unit 111 stores map data such as a three-dimensional high-precision map such as a dynamic map, a global map having less accuracy than the high-precision map but covering a large area, and a local map including information around the user's car.

The automatic driving control unit 112 performs control related to the automatic driving such as autonomous traveling or driving assist. Specifically, for example, the automatic driving control unit 112 performs cooperative control for the purpose of implementing an advanced driver assistance system (ADAS) function including collision avoidance or shock mitigation of the user's car, following travel based on a vehicular gap, vehicle speed maintaining travel, collision warning of the user's car, lane out warning of the user's car, and the like. Furthermore, for example, the automatic driving control unit 112 performs the cooperative control for the purpose of automatic driving and the like of autonomous travel without depending on an operation of the driver. The automatic driving control unit 112 includes a detection unit 131, a self-position estimation unit 132, a situation analysis unit 133, a planning unit 134, and an operation control unit 135.

The detection unit 131 detects various types of information necessary for controlling the automatic driving. The detection unit 131 includes a vehicle exterior information detection unit 141, a vehicle interior information detection unit 142, and a vehicle state detection unit 143.

The vehicle exterior information detection unit 141 performs processing of detecting information outside the user's car on the basis of data or signals from each unit of the vehicle control system 100. For example, the vehicle exterior information detection unit 141 performs detection processing, recognition processing, and tracking processing, for an object around the user's car, and processing of detecting a distance to the object. Objects to be detected include, for example, vehicles, people, obstacles, structures, roads, traffic lights, traffic signs, road markings, and the like. Furthermore, for example, the vehicle exterior information detection unit 141 performs processing of detecting an environment around the user's car. The surrounding environment to be detected includes, for example, weather, temperature, humidity, brightness, road surface condition, and the like. The vehicle exterior information detection unit 141 supplies data indicating results of the detection processing to the self-position estimation unit 132, a map analysis unit 151, a traffic rule recognition unit 152, and a situation recognition unit 153 of the situation analysis unit 133, and an emergency avoidance unit 171 and the like of the operation control unit 135.

The vehicle interior information detection unit 142 performs processing of detecting information inside the vehicle on the basis of data or signals from each unit of the vehicle control system 100. For example, the vehicle interior information detection unit 142 performs driver authentication processing and recognition processing, driver state detection processing, passenger detection processing, vehicle interior environment detection processing, and the like. The state of the driver to be detected includes, for example, a physical condition, an arousal level, a concentration level, a fatigue level, a line-of-sight direction, or the like. The environment in the vehicle to be detected includes, for example, temperature, humidity, brightness, odor, and the like. The vehicle interior information detection unit 142 supplies data indicating results of the detection processing to the situation recognition unit 153 of the situation analysis unit 133, the emergency avoidance unit 171 of the operation control unit 135, and the like.

The vehicle state detection unit 143 performs processing of detecting the state of the user's car on the basis of data or signals from each unit of the vehicle control system 100. The state of the user's car to be detected includes, for example, a speed, an acceleration, a steering angle, presence or absence of abnormality, content of abnormality, a state of driving operation, position and tilt of a power seat, a state of door lock, a state of another in-vehicle device, or the like. The vehicle state detection unit 143 supplies data indicating results of the detection processing to the situation recognition unit 153 of the situation analysis unit 133, the emergency avoidance unit 171 of the operation control unit 135, and the like.

The self-position estimation unit 132 performs processing of estimating the position, posture, and the like of the user's car on the basis of the data or signals from the units of the vehicle control system 100 such as the vehicle exterior information detection unit 141 and the situation recognition unit 153 of the situation analysis unit 133. Furthermore, the self-position estimation unit 132 generates a local map (hereinafter referred to as self-position estimation map) to be used for estimating the self-position, as needed. The self-position estimation map is a high-precision map using a technology such as simultaneous localization and mapping (SLAM), or the like. The self-position estimation unit 132 supplies data indicating a result of the estimation processing to the map analysis unit 151, the traffic rule recognition unit 152, and the situation recognition unit 153 of the situation analysis unit 133, and the like. Furthermore, the self-position estimation unit 132 causes the storage unit 111 to store the self-position estimation map.

The situation analysis unit 133 performs processing of analyzing the situation of the user's car and its surroundings. The situation analysis unit 133 includes the map analysis unit 151, the traffic rule recognition unit 152, the situation recognition unit 153, and a situation prediction unit 154.

The map analysis unit 151 performs processing of analyzing various maps stored in the storage unit 111, using the data or signals from the units of the vehicle control system 100 such as the self-position estimation unit 132 and the vehicle exterior information detection unit 141, as needed, and builds a map including information necessary for automatic driving processing. The map analysis unit 151 supplies the built map to the traffic rule recognition unit 152, the situation recognition unit 153, the situation prediction unit 154, and a route planning unit 161, an action planning unit 162, and an operation planning unit 163 of the planning unit 134, and the like.

The traffic rule recognition unit 152 performs processing of recognizing a traffic rule around the user's car on the basis of the data or signals from the units of the vehicle control system 100 such as the self-position estimation unit 132, the vehicle exterior information detection unit 141, and the map analysis unit 151. By the recognition processing, for example, the position and state of signals around the user's car, the content of traffic regulation around the user's car, a travelable lane, and the like are recognized. The traffic rule recognition unit 152 supplies data indicating a result of the recognition processing to the situation prediction unit 154 and the like.

The situation recognition unit 153 performs processing of recognizing the situation regarding the user's car on the basis of the data or signals from the units of the vehicle control system 100 such as the self-position estimation unit 132, the vehicle exterior information detection unit 141, the vehicle interior information detection unit 142, the vehicle state detection unit 143, and the map analysis unit 151. For example, the situation recognition unit 153 performs processing of recognizing a situation of the user's car, a situation around the user's car, a situation of the driver of the user's car, and the like. Furthermore, the situation recognition unit 153 generates a local map (hereinafter referred to as situation recognition map) used for recognizing the situation around the user's car, as needed. The situation recognition map is, for example, an occupancy grid map.

The situation of the user's car to be recognized includes, for example, the position, attitude, movement (for example, speed, acceleration, moving direction, and the like) of the user's car, and the presence or absence, content of abnormality, and the like. The situation around the user's car to be recognized includes, for example, types and positions of surrounding stationary objects, types of surrounding moving objects, positions and motions (for example, speed, acceleration, moving direction, and the like), configurations of surrounding roads and conditions of road surfaces, as well as surrounding weather, temperature, humidity, brightness, and the like. The state of the driver to be recognized includes, for example, physical condition, arousal level, concentration level, fatigue level, line-of-sight (line-of-sight direction) motion, driving operation, and the like.

The situation recognition unit 153 supplies the data indicating a result of the recognition processing (including the situation recognition map, as needed) to the self-position estimation unit 132, the situation prediction unit 154, and the like. Furthermore, the situation recognition unit 153 causes the storage unit 111 to store the situation recognition map.

The situation prediction unit 154 performs processing of predicting the situation regarding the user's car on the basis of the data or signals from the units of the vehicle control system 100 such as the map analysis unit 151, the traffic rule recognition unit 152, and the situation recognition unit 153. For example, the situation prediction unit 154 performs processing of predicting the situation of the user's car, the situation around the user's car, the situation of the driver, and the like.

The situation of the user's car to be predicted includes, for example, a behavior of the user's car, occurrence of abnormality, a travelable distance, and the like. The situation around the user's car to be predicted includes, for example, a behavior of a moving object around the user's car, a change in a signal state, a change in the environment such as weather, and the like. The situation of the driver to be predicted includes, for example, a behavior and physical conditions of the driver, and the like.

The situation prediction unit 154 supplies data indicating a result of the prediction processing together with the data from the traffic rule recognition unit 152 and the situation recognition unit 153 to the route planning unit 161, the action planning unit 162, the operation planning unit 163 of the planning unit 134, and the like.

The route planning unit 161 plans a route to a destination on the basis of the data or signals from the units of the vehicle control system 100 such as the map analysis unit 151 and the situation prediction unit 154. For example, the route planning unit 161 sets a route to a destination specified from a current position on the basis of the global map. Furthermore, for example, the route planning unit 161 appropriately changes the route on the basis of situations of congestion, accidents, traffic regulations, construction, and the like, the physical conditions of the driver, and the like. The route planning unit 161 supplies data indicating the planned route to the action planning unit 162 and the like.

The action planning unit 162 plans an action of the user's car for safely traveling in the route planned by the route planning unit 161 within a planned time on the basis of the data or signals from the units of the vehicle control system 100 such as the map analysis unit 151 and the situation prediction unit 154. For example, the action planning unit 162 makes a plan of starting, stopping, traveling directions (for example, forward, backward, turning left, turning right, turning, and the like), driving lane, traveling speed, passing, and the like. The action planning unit 162 supplies data indicating the planned action of the user's car to the operation planning unit 163 and the like.

The operation planning unit 163 plans an operation of the user's car for implementing the action planned by the action planning unit 162 on the basis of the data or signals from the units of the vehicle control system 100 such as the map analysis unit 151 and the situation prediction unit 154. For example, the operation planning unit 163 plans acceleration, deceleration, a traveling track, and the like. The operation planning unit 163 supplies data indicating the planned operation of the user's car to an acceleration and deceleration control unit 172 and a direction control unit 173 of the operation control unit 135, and the like.

The operation control unit 135 controls the operation of the user's car. The operation control unit 135 includes the emergency avoidance unit 171, the acceleration and deceleration control unit 172, and the direction control unit 173.

The emergency avoidance unit 171 performs processing of detecting an emergency situation such as collision, contact, entry into a dangerous zone, driver's abnormality, vehicle's abnormality, and the like on the basis of the detection results of the vehicle exterior information detection unit 141, the vehicle interior information detection unit 142, and the vehicle state detection unit 143. In a case where the emergency avoidance unit 171 detects occurrence of the emergency situation, the emergency avoidance unit 171 plans the operation of the user's car for avoiding the emergency situation, such as sudden stop or sharp turn. The emergency avoidance unit 171 supplies data indicating the planned operation of the user's car to the acceleration and deceleration control unit 172, the direction control unit 173, and the like.

The acceleration and deceleration control unit 172 performs acceleration and deceleration for implementing the operation of the user's car planned by the operation planning unit 163 or the emergency avoidance unit 171. For example, the acceleration and deceleration control unit 172 calculates a control target value of a drive force generation device or a braking device for implementing the planned acceleration, deceleration, or sudden stop, and supplies a control command indicating the calculated control target value to the drive system control unit 107.

The direction control unit 173 controls a direction for implementing the operation of the user's car planned by the operation planning unit 163 or the emergency avoidance unit 171. For example, the direction control unit 173 calculates a control target value of a steering mechanism for implementing the traveling track or sharp turn planned by the operation planning unit 163 or the emergency avoidance unit 171, and supplies a control command indicating the calculated control target value to the drive system control unit 107.

### <<4. Configuration Example of Report Recipient Terminal of Present Disclosure>>

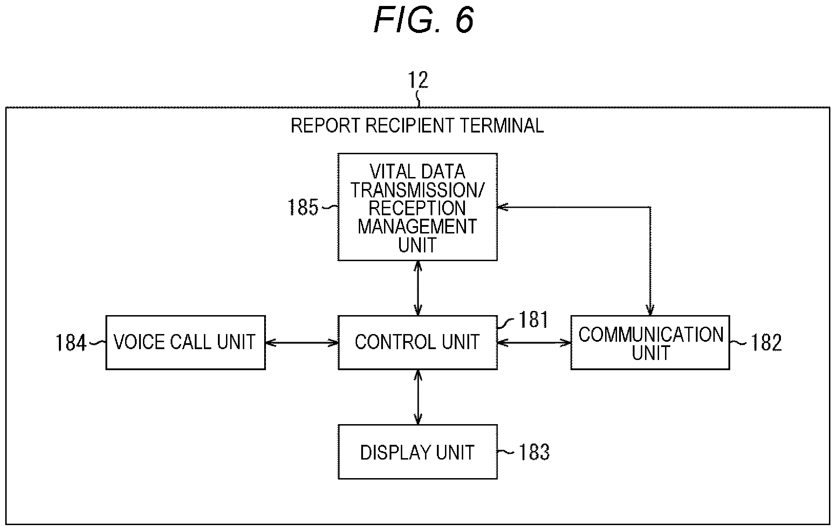

Next, a configuration example of the report recipient terminal of the present disclosure will be described with reference to the block diagram in Fig. 6.

The report recipient terminal 12 in Fig. 6 includes a control unit 181, a communication unit 182, a display unit 183, a voice call unit 184, and a vital data transmission/reception management unit 185.

The control unit 181 includes a processor, a memory, and the like, and controls the entire operation of the report recipient terminal 12.

The communication unit 182 includes an Ethernet board and the like, and exchanges data, programs, and the like with the vehicle 11 and the medical operator terminal 13 via the network 14 in Fig. 4 to implement mutual communication.

The display unit 183 includes a liquid crystal display (LCD), an organic electro luminescence (EL), and the like, and is controlled by the control unit 181 to display various types of information. Furthermore, the display unit 183 includes a touch panel and the like, receives an operation input of the report recipient, and outputs a signal corresponding to the operation input to the control unit 181.

The voice call unit 184 includes, for example, a microphone and a speaker, receives an input of a call voice uttered by the report recipient, converts the call voice into a predetermined signal, and transmits the predetermined signal to the vehicle 11 and the medical operator terminal 13 via the communication unit 182. Furthermore, the voice call unit 184 converts the predetermined signal regarding the call voice transmitted from the vehicle 11 or the medical operator terminal 13 via the communication unit 182 into a voice signal and outputs the voice signal as a voice from the speaker to have the report recipient to listen. By such an operation, the voice call unit 184 implements a call between the report recipient, and the driver or passenger of the vehicle 11 and the medical operator who operates the medical operator terminal 13.

The vital data transmission/reception management unit 185 receives the transmitted vital data and transmits the received vital data to the medical operator terminal 13 via the communication unit 182 when the vehicle 11 reports the abnormal physical condition of the driver. At this time, the vital data includes the information that specifies the driver in which the abnormal physical condition is detected and the position information of the vehicle 11. Therefore, the vital data transmission/reception management unit 185 transmits the vital data together with the information that specifies the driver and the position information of the vehicle 11 to the medical operator terminal 13.

In response to the transmission, the information of the medical institution capable of providing the medical treatment required for the driver in the abnormal physical condition and the information of the necessity of the emergency vehicle is transmitted from the medical operator terminal 13 to the report recipient terminal 12.

Here, the control unit 181 functions as an agent, for example, controls the communication unit 182, receives the information (including the position information) of the medical institution capable of providing the medical treatment, and transmits the received information to the vehicle 11. Furthermore, in a case where arrangement of the emergency vehicle is determined to be necessary on the basis of the information of the necessity of the emergency vehicle, the control unit 181 functions as an agent, and specifies an appropriate position of the merging point between the emergency vehicle and the vehicle 11 in consideration of the transportation route from the position information of the vehicle 11 and the position information of the medical institution capable of providing the medical treatment and transmits the position information of the merging point together with the information of the medical institution capable of providing the medical treatment to the vehicle 11.

### <<5. Configuration Example of Medical Operator Terminal of Present Disclosure>>

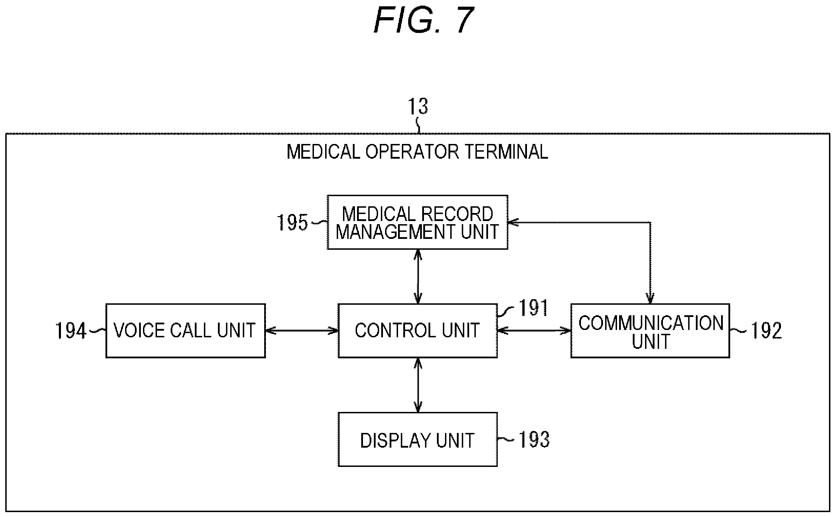

Next, a configuration example of the medical operator terminal 13 will be described with reference to Fig. 7.

The medical operator terminal 13 in Fig. 7 includes a control unit 191, a communication unit 192, a display unit 193, a voice call unit 194, and a medical record management unit 195.

The control unit 191 includes a processor, a memory, and the like, and controls the entire operation of the medical operator terminal 13.

The communication unit 192 includes an Ethernet board and the like, and exchanges data, programs, and the like with the vehicle 11 and the report recipient terminal 12 via the network 14 in Fig. 4 to implement mutual communication.

The display unit 193 includes a liquid crystal display (LCD), an organic electro luminescence (EL), and the like, and is controlled by the control unit 181 to display various types of information. Furthermore, the display unit 193 includes a touch panel and the like, receives an operation input of the medical operator, and outputs a signal corresponding to the operation input to the control unit 191.

The voice call unit 194 includes, for example, a microphone and a speaker, receives an input of a call voice uttered by the medical operator, converts the call voice into a predetermined signal, and transmits the predetermined signal to the vehicle 11 and the report recipient terminal 12 via the communication unit 182. Furthermore, the voice call unit 194 converts the predetermined signal regarding the call voice transmitted from the vehicle 11 or the report recipient terminal 12 via the communication unit 192 into a voice signal and outputs the voice signal as a voice from the speaker to have the medical operator to listen. By such an operation, the voice call unit 194 implements a call between the medical operator, and the driver or passenger of the vehicle 11 and the report recipient who operates the report recipient terminal 12.

The medical record management unit 195 receives the vital data transmitted via the communication unit 192 when the vehicle 11 reports the abnormal physical condition of the driver. At this time, the vital data includes the information that specifies the driver in which the abnormal physical condition is detected and the position information of the vehicle 11. Therefore, the medical record management unit 195 searches for electronic data of the medical record stored in advance on the basis of the information that specifies the driver in the abnormal physical condition. When detecting the medical record of the driver in the abnormal physical condition, the medical record management unit 195 outputs information of the detected medical record to the control unit 191 and causes the display unit 193 to display the medical record.

The medical operator judges medical treatment necessary for the driver in the abnormal physical condition, specifies an appropriate medical institution from a positional relationship between a medical institution capable of providing the treatment and the position information of the vehicle 11, determines necessity of the emergency vehicle, and operates the display unit 193 including a touch panel to input necessary information.

The control unit 191 controls the communication unit 192 to transmit the information of the medical institution and the information of the necessity of the emergency vehicle input to the display unit 193 to the vehicle 11 via the report recipient terminal 12.

At this time, the control unit 191 may function as an agent, and determine necessary treatment, specify the medical institution capable of providing the treatment, determine the necessity of the emergency vehicle, and make a proposal to the driver in the abnormal physical condition on behalf of the medical operator. Furthermore, the control unit 191 may function as an agent in place of a doctor and transmit the determination result as it is to the vehicle 11 via the report recipient terminal 12.

### <<6. Configuration Example of Automatic Driving Control Unit That Detects Abnormal Physical Condition on Basis of Vital Data and Implements Driving Control in Consideration of Safety>>

Next, a configuration example of the automatic driving control unit that detects the abnormal physical condition on the basis of the vital data and implements the driving control of the vehicle 11 in consideration of safety when detecting the abnormal physical condition will be described with reference to the block diagram in Fig. 8.

Note that Fig. 8 illustrates a configuration example excerpting a part of the configuration example of the vehicle control system 100 that detects the abnormal physical condition on the basis of the line-of-sight detection and the vital data and implements the driving control considering safety, in the configuration example of the functions that implement the vehicle control system 100 described with reference to Fig. 5.

The data acquisition unit 102 includes an in-vehicle sensor 231 for detecting the line-of-sight and a vital data acquisition sensor 232 for detecting the heartbeat, pulse, body surface pulse wave, and body temperature. Specifically, the in-vehicle sensor 231 referred to here corresponds to the camera Cam having the functions of the image sensor and the ToF sensor (ToF camera) described with reference to Fig. 1.

That is, the in-vehicle sensor 231 acquires an image near the face of the driver H from the image acquired by the image sensor or the distance image acquired by the ToF sensor, and outputs the image to the vehicle interior information detection unit 142 of the detection unit 131 in the automatic driving control unit 112. The vehicle interior information detection unit 142 extracts information (characteristic amount information) in an image that specifies the driver on the basis of the face image and determines the presence or absence of the line-of-sight detection of the driver H (including whether or not the line-of-sight is staring at a predetermined direction for a predetermined time or more) on the basis of the position of the pupil and the like in the image, and outputs a determination result to the situation recognition unit 153 of the situation analysis unit 133.

Furthermore, the vital data acquisition sensor 232 has a configuration corresponding to, for example, the wearable device B in Fig. 1, and detects information of the heartbeat, pulse, body surface pulse wave, and body temperature of the driver H and outputs the detection result to the vehicle interior information detection unit 142 of the detection unit 131 in the automatic driving control unit 112. The vehicle interior information detection unit 142 outputs biodata including the information of the driver's heartbeat, pulse, body surface pulse wave, and body temperature to the situation recognition unit 153 of the situation analysis unit 133.

Moreover, the vital data acquisition sensor 232 is, for example, a microwave sensor that detects the driver's heartbeat in a noncontact manner and outputs the detection result to the vehicle interior information detection unit 142 of the detection unit 131 of the automatic driving control unit 112.

The situation recognition unit 153 detects the abnormal physical condition of the driver according to the detected information that specifies the driver, the line-of-sight detection, and the vital data such as the heartbeat, pulse, body surface pulse wave, and body temperature, and implements the driving control according to the detection result. The situation recognition unit 153 includes a vehicle interior environment recognition unit 201, a feedback control unit 202, an abnormal situation processing unit 203, and an E-call control unit (E-call) 204.

The vehicle interior environment recognition unit 201 determines whether or not the abnormal physical condition is occurring in the driver in the vehicle on the basis of vehicle interior information including the presence or absence of the line-of-sight detection, the vital data such as the heartbeat, pulse, body surface pulse wave, and the body temperature, and the like supplied from the vehicle interior information detection unit 142 of the detection unit 131 according to the automatic driving level.

More specifically, the vehicle interior environment recognition unit 201 may be able to determine whether or not the abnormal physical condition is occurring in the driver on the basis of the vital data by machine learning or the like. For example, a model of making determination using an index for determining whether or not the abnormal physical condition is occurring from the presence or absence of the line-of-sight detection, the vital data such as the body temperature, heartbeat, pulse, and body surface pulse wave, and the like may be provided. Such a model can be implemented using any machine learning technology such as genome-wide association study (GWAS), deep learning, or support vector machine (SVM).

Furthermore, the vehicle interior environment recognition unit 201 may control the communication unit 103 to transmit the vital data to a specialized medical institution or the like capable of judging the presence or absence of occurrence of the abnormal physical condition, and determine whether or not the abnormal physical condition is occurring in the driver on the basis of the judgment result of the specialized medical institution or the like.

However, if exchange of information by controlling the communication unit 103 to transmit the vital data to the specialized medical institution in real time and obtaining the judgment result is repeated, there may be a time lag in the judgment or the judgment result may not be obtained if the communication state is poor.

Therefore, the vehicle interior environment recognition unit 201 may use both the determination result of the presence or absence of the abnormal physical condition of the driver made by the vehicle interior environment recognition unit 201 itself based on the machine learning and the judgment result by the specialized medical institution.

That is, the vehicle interior environment recognition unit 201 always determines the occurrence of the abnormal physical condition of the driver, but may use the judgment result of the highly reliable specialized medical institution when the communication state is good and use its own determination result when the communication state becomes poor.

Note that, hereinafter, description will be given on the assumption that the vehicle interior environment recognition unit 201 itself determines the occurrence of the abnormal physical condition of the driver on the basis of the vital data, but may determine the occurrence of the abnormal physical condition of the driver by any of the above-described methods.

Furthermore, the vehicle interior environment recognition unit 201 outputs the determination result to the situation prediction unit 154 and the feedback control unit 202.

More specifically, in a case where the automatic driving level is the level 4 or 5, the automatic driving can be continued even in the inattentive driving or dozing driving, so the vehicle interior environment recognition unit 201 does not detect the line-of-sight for determining the inattentive driving or dozing driving, and detects the vital data such as the heartbeat, pulse, body surface pulse wave, and body temperature and supplies the determination result to the situation prediction unit 154 in the a where there is no abnormality.

The situation prediction unit 154 does not add a change to the operation plan being executed by the planning unit 134 and allows the current automatic driving to continue because no change is predicted in the situation as long as the determination result indicating no abnormality is supplied.

Furthermore, in a case where the automatic driving level is the level 4 or 5, it is immediately determined that the abnormal physical condition is occurring in the driver when abnormality is detected in the vital data.

Moreover, in a case where the automatic driving level is the level 3 or a lower level, the automatic driving cannot be continued in the inattentive driving or dozing driving, so the presence or absence of the abnormal physical condition is determined including the line-of-sight detection result by which the inattentive driving or dozing driving can be determined in addition to the vital data.

More specifically, the vehicle interior environment recognition unit 201 determines the presence or absence of the abnormal physical condition of the driver on the basis of whether or not the line-of-sight acquired by the in-vehicle sensor 231 cannot be detected by the detection unit 131 (for example, there is no change in the line-of-sight direction for a predetermined time or more or the pupil is open), and whether or not there is abnormality in the heartbeat, pulse, body surface pulse wave, and body temperature as the vital data detected by the vital data acquisition sensor 232.

Then, the vehicle interior environment recognition unit 201 determines the presence or absence of abnormality of the driver on the basis of the line-of-sight detection and the vital data such as the heartbeat, pulse, body surface pulse wave, and body temperature supplied from the detection unit 131, and controls the feedback control unit 202 to give a feedback that encourages arousal to the driver if there is the abnormality.

That is, even when there is no abnormality in any of the heartbeat, pulse, body surface pulse wave, body temperature, and the like but when the line-of-sight direction is unchanged, the pupil is open, or the like, there is a possibility of inattentive driving, dozing driving, or the like. In a case where the abnormal physical condition is the inattentive driving or dozing driving, as described above, the abnormality can be resolved by awakening the driver.

Therefore, the feedback control unit 202 causes the output unit 106 to give a feedback that encourages arousal to the driver via the output control unit 105 on the basis of the determination result supplied from the vehicle interior environment recognition unit 201.

More specifically, the feedback control unit 202 gives the feedback that encourages arousal by controlling the irritating odor generation unit 251 of the output unit 106 via the output control unit 105 to generate an irritating odor and irritate a sense of smell of the driver.

Furthermore, the feedback control unit 202 gives the feedback to encourage arousal of the driver by controlling the information display unit 252 of the output unit 106 to present image information (including text, moving image, and the like) that encourages arousal via the output control unit 105.

Moreover, the feedback control unit 202 gives the feedback to encourage arousal of the driver by controlling the speaker 253 of the output unit 106 to output sound via the output control unit 105.

Furthermore, the feedback control unit 202 gives the feedback to encourage arousal of consciousness by functioning as a voice agent and asking a question to the driver and causing the driver to answer the question. For example, the feedback control unit 202 gives the feedback to encourage arousal by functioning as a voice agent and implementing a dialog of asking the name and date of birth of the driver and causing the driver to answer the question, and presenting whether or not the answer is appropriate.

The feedback control unit 202 gives the feedback of encouraging arousal to the driver by at least one of the irritating odor generation unit 251, the information display unit 252, the speaker 253, or the dialog by the voice agent function.

Note that the feedback may be given not only by the irritating odor generated by the irritating odor generation unit 251, the image displayed by the information display unit 252, the sound output by the speaker 253, and the dialog by the voice agent function, but also by another method as long as information for encouraging the driver to awaken can be presented.

More specific configurations for giving the feedback include, for example, a head up display (HUD) that displays information encouraging the driver to awaken, a configuration that vibrates the seat belt, a configuration that vibrates the steering wheel, a configuration that vibrates the seat, and the like.

In a case where an abnormal state continues even if the feedback control unit 202 gives the feedback to encourage the driver to awaken, the vehicle interior environment recognition unit 201 outputs information indicating that the physical condition of the driver is abnormal as a determination result to the abnormal situation processing unit 203.

When occurrence of the abnormal physical condition of the driver is notified, the abnormal situation processing unit 203 implements the processing considering safety according to the automatic driving level.

More specifically, in a case where the automatic driving level is the level 4 or 5, the abnormal situation processing unit 203 controls the communication unit 103 to transmit the position information (self-position) of the vehicle 11 acquired from the self-position estimation unit 132, the vital data of the driver, the information that identifies the driver, and information that reports that the abnormal physical condition is occurring in the driver to the report recipient terminal 12.

Thereby, the report recipient terminal 12 receives the position information of the vehicle 11, the vital data, and the information that identifies the driver together with the report indicating that the abnormal physical condition is occurring in the driver. The report recipient terminal 12 transmits the position information of the vehicle 11, the vital data, and the information that identifies the driver to the medical operator terminal 13.

When the medical operator terminal 13 receives the position information of the vehicle 11, the vital data, and the information that identifies the driver, the medical operator terminal 13 searches for the medical record stored in advance on the basis of the information that identifies the driver.

In a case where the medical operator terminal 13 can find the medical record of the driver in the abnormal physical condition, the medical operator terminal 13 presents information of the found medical record together with the vital data to the medical operator and requests the medical operator to judge the medical treatment necessary for the driver in the abnormal physical condition and specify the medical institution capable of providing the treatment.

In a case where the medical operator terminal 13 cannot find the medical record of the driver in the abnormal physical condition, the medical operator terminal 13 presents the vital data to the medical operator and requests the medical operator to judge the medical treatment necessary for the driver in the abnormal physical condition and specify the medical institution capable of providing the treatment.

In response to the request, the medical operator judges the medical treatment necessary for the driver in the abnormal physical condition, specifies the medical institution capable of providing the treatment, judges the necessity of the rescue by the emergency vehicle, and inputs the judgment result to the medical operator terminal 13.

Here, since the vehicle 11, the report recipient terminal 12, and the medical operator terminal 13 can call one another, the driver or the passenger of the vehicle 11, the report recipient, and the medical operator make a call if possible. When more information can be acquired from the call, the medical operator can judge the medical treatment necessary for the driver in the abnormal physical condition in consideration of the call content.

Furthermore, in a case where a request is notified from the driver or the passenger of the vehicle 11 via the communication unit 103, or in a case where stop of the heartbeat or pulse, abnormal body surface pulse wave, or abnormal body temperature is confirmed on the basis of the vital data, or a large amount of bleeding is confirmed on the basis of the image of the vicinity of the face as the information for identifying the driver, the abnormal situation processing unit 203 makes an emergency call to the police station, emergency medical institution, or the like via the report recipient terminal 12 regardless of the presence or absence of input information from the medical operator.

The medical operator terminal 13 transmits the information of the medical institution capable of providing the treatment and the information of the necessity of the rescue by the emergency vehicle input by the medical operator to the report recipient terminal 12.

Note that the judgment of the medical treatment necessary for the driver in the abnormal physical condition, specification of the medical institution capable of providing the treatment, and judgment of the necessity of transportation by the emergency vehicle made by the medical operator may be implemented by an agent function by configuring the agent function by machine learning using the vital data, the medical record information, and the call content in the medical operator terminal 13.

In such a case, the agent function may determine the medical treatment necessary for the driver in the abnormal physical condition from the medical record information (in a case where the medical record is found), the vital data, the call content, and the like, and specify the medical institution capable of providing the treatment, and propose and determine the necessity of the rescue by the emergency vehicle.

Then, a proposal result and a determination result by the agent function may be transmitted to the report recipient terminal 12 as the information of the medical institution capable of providing the treatment and the information of the necessity of the rescue by the emergency vehicle input by the medical operator.

The report recipient terminal 12 receives the information of the medical institution capable of providing the treatment and the information of the necessity of transportation by the emergency vehicle.

Here, in a case where the rescue by the emergency vehicle is unnecessary, the report recipient terminal 12 specifies the position information of the medical institution on the basis of the information of the medical institution capable of providing the treatment and transmits the position information to the vehicle 11.

Furthermore, in a case where the rescue by the emergency vehicle is necessary, the report recipient terminal 12 arranges the emergency vehicle, specifies the position information of the medical institution on the basis of the information of the medical institution capable of providing the treatment, collates the position information with the position information of the vehicle 11, and specifies the optimum merging point with the emergency vehicle considering the transportation route. Then, the report recipient terminal 12 transmits the position information of the medical institution and the position information of the optimum merging point with the specified emergency vehicle to the vehicle 11.

Here, the abnormal situation processing unit 203 of the vehicle 11 controls the communication unit 103 to acquire the position information of the medical institution (the position information of the optimum merging point with the emergency vehicle) transmitted from the report recipient terminal 12. Then, the abnormal situation processing unit 203 outputs the information indicating the occurrence of the abnormal physical condition of the driver, the position information of the medical institution (or information including the position information of the optimum merging point with the emergency vehicle if the optimum merging point with the emergency vehicle is included) to the situation prediction unit 154.

The situation prediction unit 154 predicts that transportation to the medical institution is necessary from the occurrence of the abnormal physical condition of the driver on the basis of the position information of the medical institution (and the optimum merging point with the emergency vehicle) and outputs a command according to a prediction result to the operation planning unit 163 of the planning unit 134.

More specifically, the situation prediction unit 154 predicts the possibility that the abnormal physical condition is occurring in the driver, and transmits a command that implements the driving control based on the prediction result to the planning unit 134.

The operation planning unit 163 of the planning unit 134 plans the operation for implementing the driving control according to the automatic driving level when the command for implementing the driving control based on the prediction result is transmitted from the situation prediction unit 154.

More specifically, since the driving is fully automatic driving in a case where the automatic driving level is the level 5, the operation planning unit 163 plans the operation for transportation to the position information of the medical institution (or the position information of the merging point with the emergency vehicle in a case where there is the position information of the merging point with the emergency vehicle).

The operation planning unit 163 supplies the planned operation plan to the vehicle control unit 211 of the operation control unit 135.

The vehicle control unit 211 controls the acceleration and deceleration control unit 172 and the direction control unit 173 to control the operation of the vehicle 11 according to the operation plan and move the vehicle 11 to the medical institution or the merging point with the emergency vehicle.

Furthermore, since the driver is necessary according to the position or environment of traveling in a case where the automatic driving level is the level 4, processing differs depending on whether or not replacement of the driver is necessary and the replacement is possible.

That is, when the replacement of the driver is necessary and the replacement is possible when the vehicle 11 is moved to the medical institution or the merging point with the emergency vehicle, the operation planning unit 163 presents the position information of the medical institution or the position information of the merging point with the emergency vehicle to the information display unit 252 of the output unit 106 to present information for urging a driver to be replaced to drive the vehicle 11.

In this case, the replaced driver drives the vehicle 11 and moves to the medical institution or the merging point with the emergency vehicle.

Furthermore, when the replacement of the driver is necessary but the replacement is not possible when the vehicle 11 is moved to the medical institution or the merging point with the emergency vehicle, the automatic driving cannot be continued, so the operation planning unit 163 stops the vehicle 11 at a safe place.

Furthermore, the abnormal situation processing unit 203 controls the communication unit 103 to request the report recipient terminal 12 to arrange the rescue by the emergency vehicle because the driver cannot be replaced.

At this time, the report recipient terminal 12 transmits the position information of the vehicle 11 to the emergency vehicle and instructs the emergency vehicle to go to the site where the vehicle 11 is stopped.

Then, the driver in the abnormal physical condition waits for the arrival of the emergency vehicle and is transported by the emergency vehicle.

Note that, in a case where the automatic driving level is the level 4, the replacement of the driver is unnecessary depending on the traveling environment, and in a case where the automatic driving can be continued, the processing is similar to the case where the automatic driving level is the level 5.

Moreover, in a case where the automatic driving level is the level 3, the automatic driving cannot be continued when the driver cannot perform driving. Therefore, the abnormal situation processing unit 203 causes the operation planning unit 134 to transmit the command for stopping the vehicle 11 at a safe place via the situation prediction unit 154, before reporting the abnormal physical condition of the driver to the report recipient terminal 12.

With the command, the operation planning unit 163 stops the vehicle 11 at a safe place.

Subsequent processing is similar to the case where the automatic driving level is the level 4.

Furthermore, in a case where the automatic driving level is one of the levels 0 to 2, the automatic driving itself cannot be continued, and the vehicle 11 cannot be stopped at a safe place. Therefore, the abnormal situation processing unit 203 causes the operation planning unit 134 to transmit the command for stopping the vehicle 11 on the driving lane while turning on the hazard lights via the situation prediction unit 154, before reporting the abnormal physical condition of the driver to the report recipient terminal 12.

With the command, the operation planning unit 163 turns on the hazard lights and stops the vehicle 11 on the driving lane.

Subsequent processing is similar to the case where the automatic driving level is the level 4.

When information of an impact or the like detected when a collision accident occurs is detected by the detection unit 131, the E-call control unit 204 acquires information of the self-position by the self-position estimation unit 132 and controls the communication unit 103 to make an emergency call indicating that the accident has occurred including the information of the self-position to a center that reports occurrence of an accident to the police station, hospitals, and the like.

Note that there is a possibility that two emergency calls are made in one accident when the abnormal situation processing unit 203 makes an emergency call and the E-call control unit 204 reports the occurrence of the accident. In preparation for such a case, it is desirable to include identification information by which two emergency calls being the same emergency call issued from the same vehicle 11 can be recognized.

With the configuration, even if a plurality of emergency calls is issued, the center that receives the emergency calls can confirm that the emergency calls are the same emergency call transmitted from the same vehicle 11 on the basis of the identification information included in the emergency calls.

Alternatively, the abnormal situation processing unit 203 and the E-call control unit 204 may cooperate and transmit only one of the reports to the center.

In either case, it is possible to suppress waste that a plurality of institutions (the police station, medical institution, and the like) responds to one accident that has occurred in the same vehicle 11 as a plurality of emergency calls, accordingly.

### <<7. Automatic Driving Control Processing>>

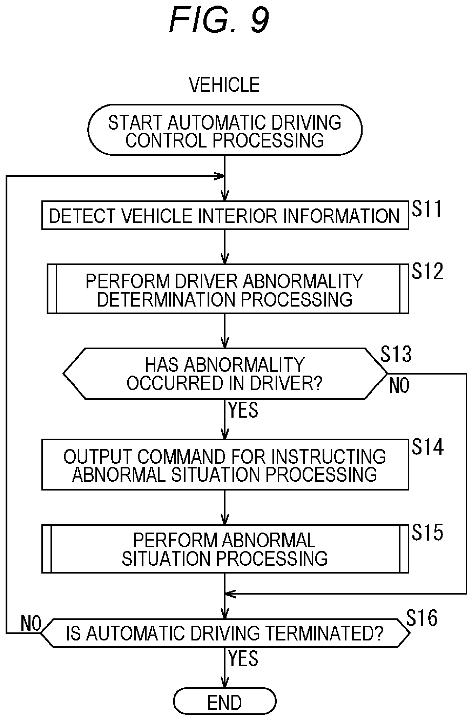

Next, automatic driving control processing by the vehicle 11 in Fig. 8 will be described with reference to the flowchart in Fig. 9.

In step S11, the vehicle interior information detection unit 142 outputs the detection results of the in-vehicle sensor 231 and the vital data acquisition sensor 232 of the data acquisition unit 102 as the vehicle interior information to the vehicle interior environment recognition unit 201 of the situation recognition unit 153.

That is, the in-vehicle sensor 231 of the data acquisition unit 102 captures an image of a vicinity of the driver's face and outputs the captured image to the vehicle interior information detection unit 142 of the detection unit 131. The vehicle interior information detection unit 142 detects the driver's line-of-sight on the basis of the position of a driver's pupil in the captured image, and outputs the detected line-of-sight together with the image of the vicinity of the face as the vehicle interior information to the vehicle interior environment recognition unit 201 in the situation recognition unit 153.

More specifically, the vehicle interior information detection unit 142 detects the position of the pupil of the driver on the basis of either the image captured by the image sensor or the distance image captured by the ToF sensor of the in-vehicle sensor 231, for example, and detects the line-of-sight of the driver according to the position of the pupil or a gaze time.

Furthermore, the vehicle interior information detection unit 142 may, for example, detect the line-of-sight from the image captured by the image sensor of the in-vehicle sensor 231 in a bright environment during daytime, and detect the line-of-sight on the basis of the distance image captured by the ToF sensor in a dark environment at night or in stormy weather, for example. Note that, by combining the image sensor and the ToF sensor, the robustness may be improved or the processing time may be shortened.

Furthermore, the vital data acquisition sensor 232 includes, for example, the wearable device B in Fig. 1, a microwave sensor, a warmth sensor, and the like, and acquires the driver's heartbeat, pulse, body surface pulse wave, and body temperature as vital data and outputs the vital data to the vehicle interior information detection unit 142 of the detection unit 131. The vehicle interior information detection unit 142 outputs the detection results of the vital data such as the heartbeat, pulse, body surface pulse wave, and body temperature as the vehicle interior information to the vehicle interior environment recognition unit 201 of the situation recognition unit 153.

In step S12, the vehicle interior environment recognition unit 201 of the situation recognition unit 153 in the situation analysis unit 133 executes driver abnormality determination processing on the basis of the vehicle interior information including the line-of-sight detection result and the vital data such as the heartbeat, pulse, body surface pulse wave, and body temperature, and determines the presence or absence of the abnormal physical condition of the driver.

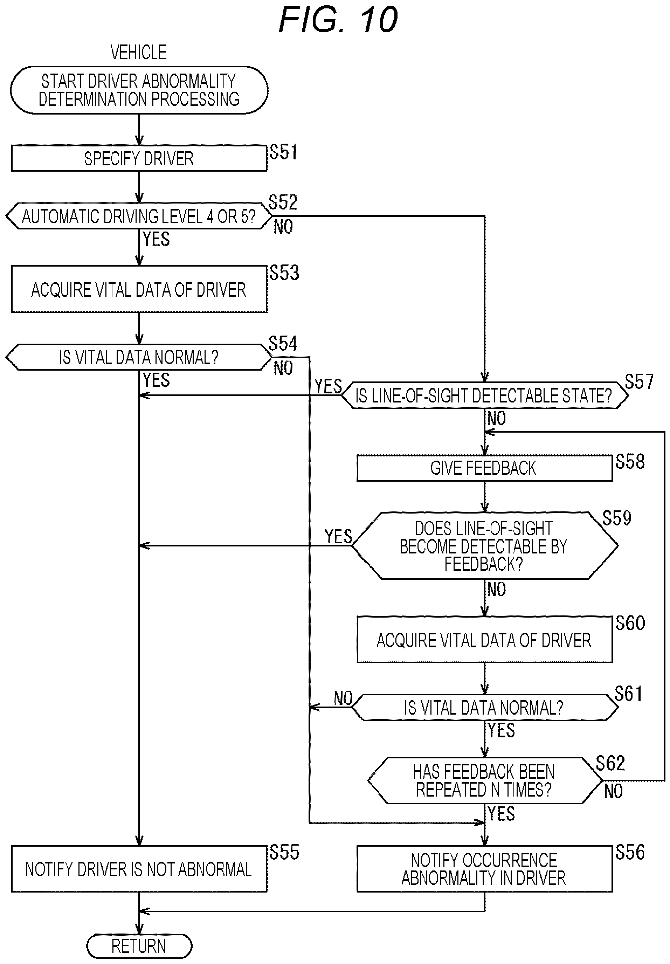

Note that details of the driver abnormality determination processing will be described below with reference to the flowchart in Fig. 10.

In step S13, the vehicle interior environment recognition unit 201 determines whether or not the abnormal physical condition is occurring in the driver on the basis of a processing result of the driver abnormality determination processing.

In step S13, in a case where it is determined that the abnormal physical condition of the driver is occurring, the processing proceeds to step S14. Note that, in a case where it is determined that the abnormal physical condition is not occurring, the processing in step S14 is skipped.

In step S14, the vehicle interior environment recognition unit 201 instructs the abnormal situation processing unit 203 to execute the abnormal situation processing.

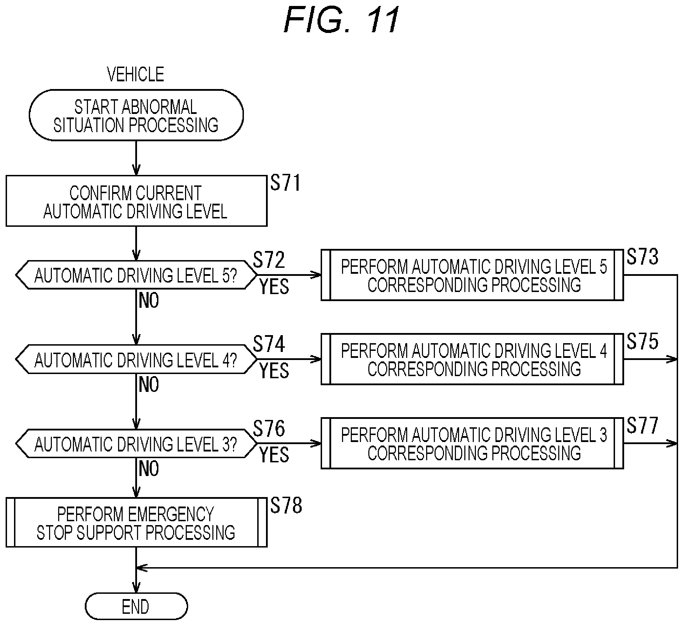

In step S15, the abnormal situation processing unit 203 executes the abnormal situation processing, and executes the driving control in the state where the abnormal physical condition has occurred in the driver. Note that details of the abnormal situation processing will be described below with reference to the flowcharts in Figs. 11 to 15.

In step S16, the vehicle interior environment recognition unit 201 determines whether or not the automatic driving is to be terminated, and the processing returns to step S11 and the subsequent processing is repeated in a case where the automatic driving is not terminated. Then, in step S16, the process is terminated when it is determined that the automatic driving is to be terminated.

By the above series of processing, the line-of-sight and the vital data of the driver are detected as the vehicle interior information, the presence or absence of the abnormal physical condition of the driver is determined on the basis of the vehicle interior information, the abnormal situation processing is executed when the abnormal physical condition is detected, and the driving control in consideration of safety is performed.

As a result, even if the driver in driving falls in an emergency situation where the abnormal physical condition such as losing consciousness occurs, for example, the driving control in consideration of safety for the driver and passengers, and surrounding vehicles and passersby can be implemented.

### <<8. Driver Abnormality Determination Processing>>

Next, the driver abnormality determination processing will be described with reference to the flowchart in Fig. 10.

In step S51, the vehicle interior environment recognition unit 201 specifies the driver on the basis of the image of the vicinity of the face in the acquired vehicle interior information. The driver's information may be the personal information registered in advance in association with the face image or may be the biometric information itself such as a fingerprint or a retinal pattern, other than the face image.

In step S52, the vehicle interior environment recognition unit 201 determines whether or not the automatic driving level is either the level 4 or the level 5, and the processing proceeds to step S53 in a case where the automatic driving level is either the level 4 or the level 5.

In step S53, the vehicle interior environment recognition unit 201 acquires the vital data such as the driver's heartbeat, pulse, body surface pulse wave, and body temperature in the acquired vehicle interior information.

In step S54, the vehicle interior environment recognition unit 201 determines whether or not the acquired vital data is normal. The determination here may be uniquely made by the vehicle interior environment recognition unit 201 using an agent implemented by machine learning on the basis of the vital data, or the vital data may be transmitted to a specialized medical institution and a determination result of the specialized medical institution may be referred to, or determination results of both the agent and the specialized medical institution may be used.

In step S54, in a case where the vital data is not abnormal, the processing proceeds to step S55.

In step S55, the vehicle interior environment recognition unit 201 notifies that the driver is not in the abnormal physical condition.

In step S54, in a case where the vital data is abnormal, the processing proceeds to step S56.

In step S56, the vehicle interior environment recognition unit 201 notifies that the driver is in the abnormal physical condition.

Furthermore, in step S52, in a case where the automatic driving level is neither the level 4 nor the level 5, that is, in a case where the automatic driving level is one of the levels 0 to 3, the processing proceeds to step S57.

In step S57, the vehicle interior environment recognition unit 201 reads out the line-of-sight detection result from the vehicle interior information and determines whether or not the line-of-sight has been detected. In step S57, in a case where the line-of-sight has been detected, it is determined that the abnormal physical condition is not occurring in the driver, and the processing proceeds to step S55.

Furthermore, in step S57, in a case where the line-of-sight has not been detected, that is, the inattentive driving or dozing driving is suspected and some sort of abnormality is suspected in the physical condition of the driver, the processing proceeds to step S58.

In step S58, the vehicle interior environment recognition unit 201 instructs the feedback control unit 202 to give a feedback to the driver. The feedback control unit 202 gives the feedback that encourages arousal to the driver from the state where the line-of-sight cannot be detected such as the inattentive driving or dozing driving by at least one of the irritating odor generation unit 251, the information display unit 252, the speaker 253 of the output unit 106, or the voice agent function via the output control unit 105.

In step S59, the vehicle interior environment recognition unit 201 acquires the vehicle interior information again, and determines whether or not the driver's arousal is encouraged by the feedback and the line-of-sight becomes able to be detected.

In step S59, in a case where it is determined that the line-of-sight becomes able to be detected, it is determined that the abnormal physical condition of the driver is not occurring, and the processing proceeds to step S55.

On the other hand, in step S59, in a case where it is determined that the state in which the line-of-sight cannot be detected continues, the processing proceeds to step S60.

In step S60, the vehicle interior environment recognition unit 201 acquires the vital data such as the driver's heartbeat, pulse, body surface pulse wave, and body temperature in the vehicle interior information.

In step S61, the vehicle interior environment recognition unit 201 determines whether or not the vital data is abnormal. In step S61, in a case where the heartbeat, pulse, body surface pulse wave, the body temperature, and the like as the vital data are abnormal, the driver is determined to be in the abnormal physical condition, and the processing proceeds to step S56.

Furthermore, in step S61, in a case where the vehicle interior environment recognition unit 201 determines that the vital data is not abnormal, the processing proceeds to step S62.

In step S62, the vehicle interior environment recognition unit 201 determines whether or not the series of processing in steps S58 to S62 has been repeated N times. In a case where the feedback has not been repeated N times, the processing proceeds to step S58. That is, when the vital data is not abnormal and the line-of-sight is not detected, the feedback is repeated by repeating the processing in steps S58 to S61 until the line-of-sight is detected. The situation where the series of processing in steps S58 to S62 is repeated is a situation in which the state where the line-of-sight cannot be detected continues despite the feedback is repeatedly given, but the state where the vital data is not abnormal continues.

In such a case, there is a possibility that the vital data is not abnormal but the inattentive driving or dozing driving is continuing. Therefore, it is determined that the abnormal physical condition is occurring in the driver, which cannot be determined from the vital data, and the processing proceeds to step S56.

By the above processing, in a case where the automatic driving level is the level 4 or the level 5, it is determined that the abnormal physical condition is not occurring in the driver unless the vital data is abnormal.

Furthermore, in a case where the automatic driving level is one of the levels 0 to 3, it is determined that the abnormal physical condition is not occurring in the driver when the driver is present and is not performing the inattentive driving or dozing driving, that is, the line-of-sight is detected. Here, when the driver is performing the inattentive driving or dozing driving, that is, the line-of-sight is not detected, the feedback is given to encourage the driver to awaken. When the line-of-sight becomes detected by the feedback, it is determined that the abnormal physical condition is not occurring in the driver.

Moreover, it is determined that the abnormal physical condition is occurring in the driver in all of the following situations: where the vital data is abnormal in a case where the automatic driving level is the level 4 or the level 5; where the driver is present and is performing the inattentive driving or dozing driving, the line-of-sight is not detected by arousal even if the feedback is given, and the vital data is abnormal; and where the line-of-sight is not detected but the state where the vital data is not abnormal is repeated N times even when the feedback is repeatedly given in a case where the automatic driving level is one of the levels 0 to 3.

### <<9. Abnormal Situation Processing>>

Next, the abnormal situation processing will be described with reference to the flowchart in Fig. 11. The abnormal situation processing is executed when it is determined that the abnormal physical condition is occurring by the driver abnormality determination processing.

That is, in step S71, the abnormal situation processing unit 203 confirms the current automatic driving level.

In step S72, the abnormal situation processing unit 203 determines whether or not the current automatic driving level is the level 5.

In step S72, in a case where the automatic driving level is the level 5, the processing proceeds to step S73.

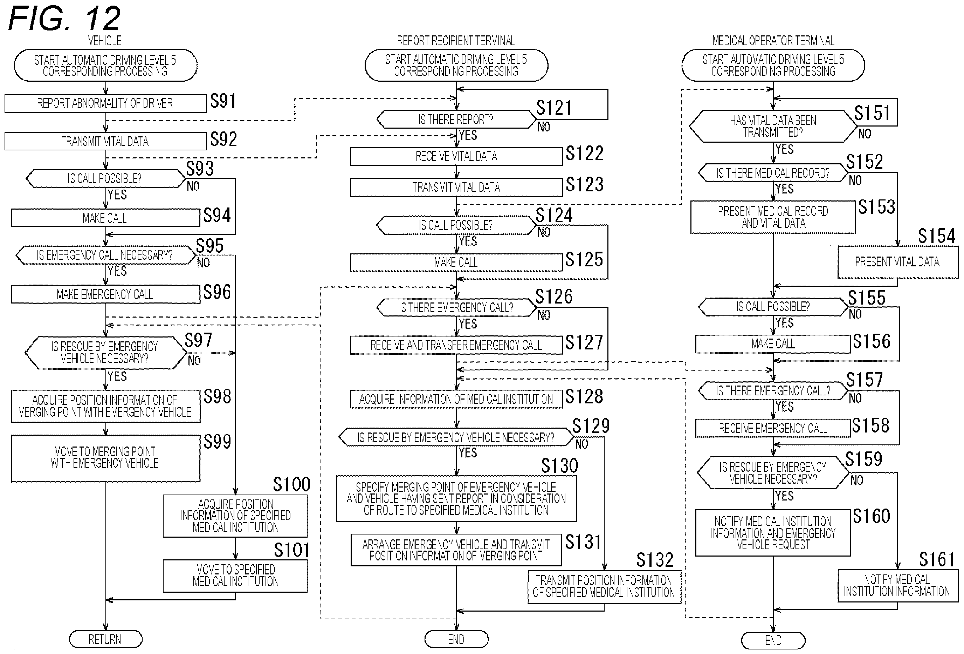

In step S73, the abnormal situation processing unit 203 executes automatic driving level 5 corresponding processing to control the driving of the vehicle 11. Note that details of the automatic driving level 5 corresponding processing will be described later with reference to the flowchart in Fig. 12.

In step S73, in a case where the automatic driving level is not the level 5, the processing proceeds to step S74.

In step S74, the abnormal situation processing unit 203 determines whether or not the current automatic driving level is the level 4.

In step S74, in a case where the automatic driving level is the level 4, the processing proceeds to step S75.

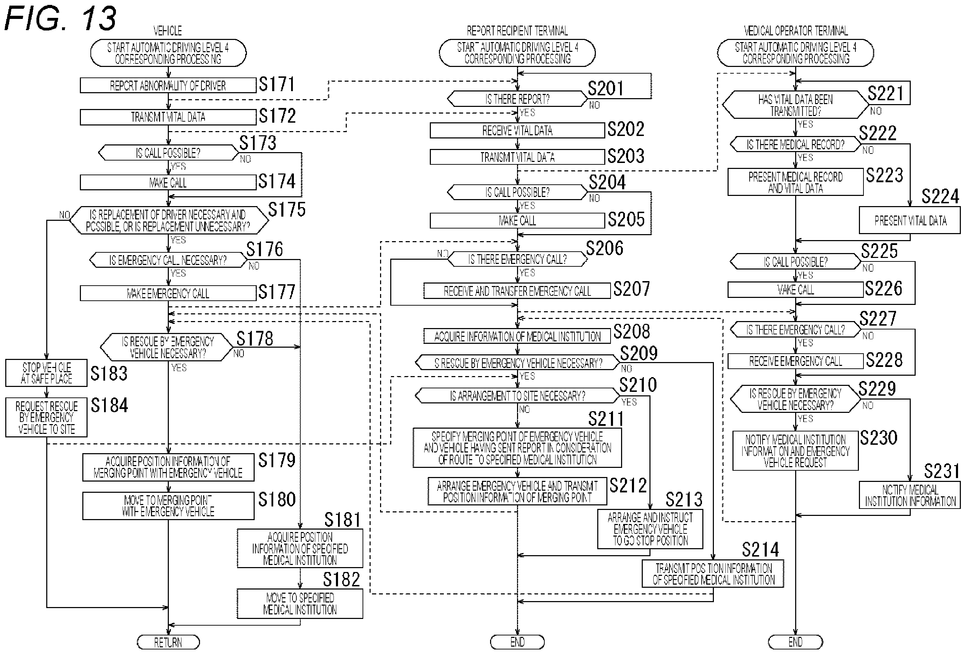

In step S75, the abnormal situation processing unit 203 executes automatic driving level 4 corresponding processing to control the driving of the vehicle 11. Note that details of the automatic driving level 4 corresponding processing will be described later with reference to the flowchart in Fig. 13

In step S74, in a case where the automatic driving level is not the level 4, the processing proceeds to step S76.

In step S76, the abnormal situation processing unit 203 determines whether or not the current automatic driving level is the level 3.

In step S76, in a case where the automatic driving level is the level 3, the processing proceeds to step S77.

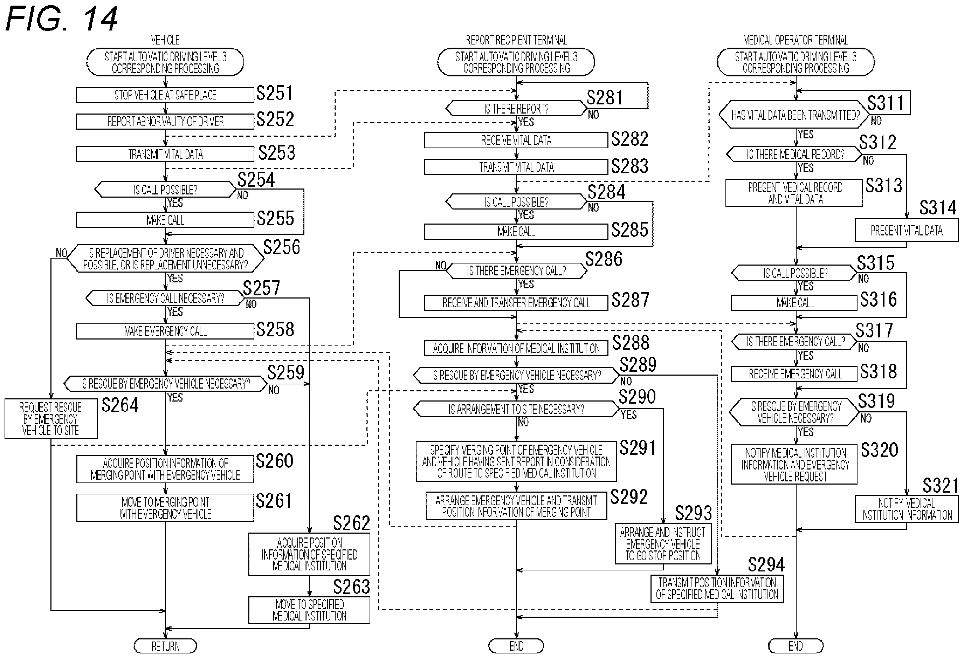

In step S77, the abnormal situation processing unit 203 executes automatic driving level 3 corresponding processing to control the driving of the vehicle 11. Note that details of the automatic driving level 3 corresponding processing will be described later with reference to the flowchart in Fig. 14

In step S76, in a case where the automatic driving level is not the level 3, that is, the automatic driving level is not one of the levels 3 to 5, the automatic driving level is determined to be the level 2 or a lower level and the processing proceeds to step S78.

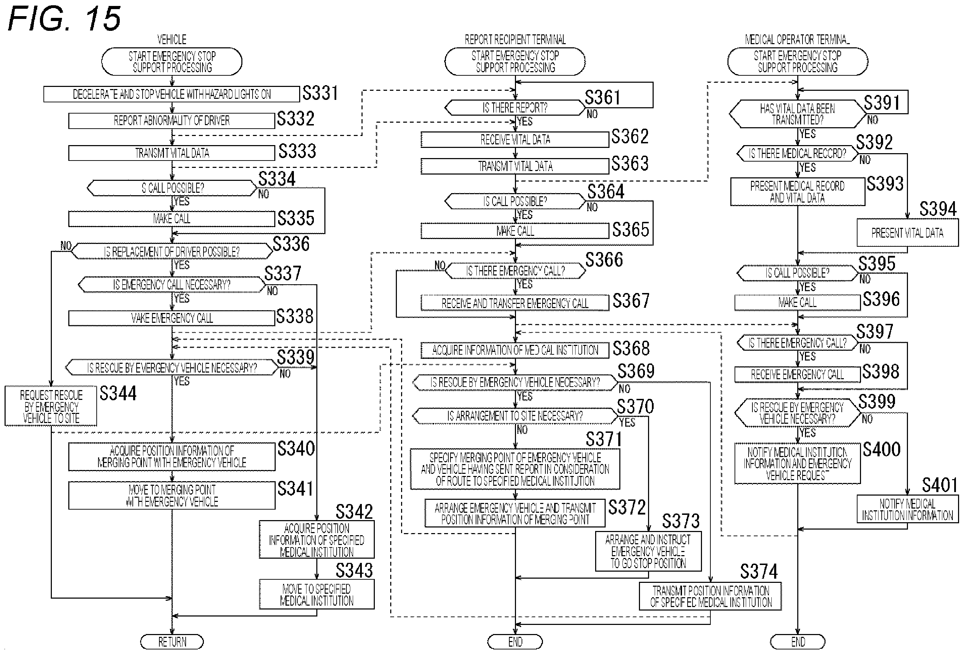

In step S78, the abnormal situation processing unit 203 executes emergency stop support processing to control the driving of the vehicle 11. Note that details of the emergency stop support processing will be described below with reference to the flowchart in Fig. 15.

By the above processing, in a case where the automatic driving level is one of the levels 5 to 3, the automatic driving level 5 corresponding processing, the automatic driving level 4 corresponding processing, or the automatic driving level 3 corresponding processing is performed, and in a case where the automatic driving level is one of the levels 0 to 2, the emergency stop support processing is performed. That is, the abnormal situation processing according to the automatic driving level is performed, and the processing for implementing the driving control in consideration of safety for the driver and passengers, and the surrounding vehicles, passersby, and the like is performed.

### <<10. Automatic Driving Level 5 Corresponding Processing>>

Next, the automatic driving level 5 corresponding processing will be described with reference to the flowchart in Fig. 12. Note that the following processing is cooperatively performed by the transportation system 1 in Fig. 4, which includes the vehicle 11, the report recipient terminal 12, and the medical operator terminal 13.

That is, in step S91, the abnormal situation processing unit 203 of the vehicle 11 controls the communication unit 103 to transmit a report indicating that the abnormal physical condition has occurred in the driver to the report recipient terminal 12.

In response to the report, in step S121, in the report recipient terminal 12, the control unit 181 controls the communication unit 182 to determine whether or not the report indicating that the abnormal physical condition has occurred in the driver has been transmitted from the vehicle 11 and repeats similar processing until the report is transmitted.

Then, in step S121, when the report indicating that the abnormal physical condition has occurred in the driver is transmitted, the process proceeds to step S122.

Furthermore, in step S92, in the vehicle 11, the abnormal situation processing unit 203 controls the communication unit 103 to transmit the information that identifies the driver including the driver's face image, the position information of the vehicle 11, and the vital data to the report recipient terminal 12.

In response to the transmission, in step S122, in the report recipient terminal 12, the control unit 181 controls the vital data transmission/reception management unit 185 to receive the information that identifies the driver including the driver's face image and the vital data including the position information of the vehicle 11 transmitted from the vehicle 11 via the communication unit 182.

In step S123, the control unit 181 controls the vital data transmission/reception management unit 185 to transmit the information that identifies the driver including the driver's face image and the vital data including the position information of the vehicle 11 transmitted from the vehicle 11 to the medical operator terminal 13 via the communication unit 182.

Moreover, in step S151, in the medical operator terminal 13, the medical record management unit 195 controls the communication unit 192 to determine whether or not the information that identifies the driver and the vital data including the position information of the vehicle 11 have been transmitted from the report recipient terminal 12, and repeats similar processing until the information is transmitted.

In step S151, in a case where the information that identifies the driver and the vital data including the position information of the vehicle 11 have been transmitted, the process proceeds to step S152.

In step S152, the medical record management unit 195 receives the information that identifies the driver and the vital data including the position information of the vehicle 11, searches for the medical record on the basis of the information that identifies the driver, and determines whether or not the medical record of the driver in the abnormal physical condition is registered in advance.

In step S152, in a case where the medical record of the driver in the abnormal physical condition is registered, the processing proceeds to step S153.

In step S153, the control unit 191 presents the transmitted vital data together with the registered medical record information to the display unit 193.

On the other hand, in step S152, in a case where the medical record is not registered, the processing proceeds to step S154.

In step S154, the control unit 191 presents the transmitted vital data to the display unit 193.

By the processing, the information of the vital data of the driver in the abnormal physical condition is displayed on the display unit 193, and the medical record information is displayed together in a case where the medical record is present, whereby the medical operator can judge the treatment to be applied to the driver in the abnormal physical condition.

Furthermore, in step S93, in the vehicle 11, the abnormal situation processing unit 203 determines whether or not at least either the driver or the passenger can make a call with the report recipient of the report recipient terminal 12 and the medical operator of the medical operator terminal 13 on the basis of the vehicle interior information.

That is, the abnormal situation processing unit 203 determines whether or not a call is possible on the basis of the image and the vital data of the driver or the passenger detected by the various sensors of the data acquisition unit 102, for example, on the basis of whether or not the driver or the passenger is conscious of being able to make a call or whether or not the driver or the passenger can make an appropriate response to a question.

In step S93, in a case where it is determined that a call is possible, the processing proceeds to step S94.

In step S94, the abnormal situation processing unit 203 controls the communication unit 103 to request a connection for implementing a call with the report recipient terminal 12 and the medical operator terminal 13, and sets a communication state in which the call is possible.

Furthermore, the abnormal situation processing unit 203 is set to be able to accept an input of a call voice of the driver or the passenger input through the microphone in the data acquisition unit 102.

Furthermore, the abnormal situation processing unit 203 is set to be able to output the call voice transmitted from the report recipient terminal 12 and the medical operator terminal 13 through the speaker 253 of the output unit 106.

Here, in step S124, in the report recipient terminal 12, the control unit 181 controls the communication unit 182 to determine whether or not a call is possible in the vehicle 11 and there is a connection request for implementing the call. In step S124, in a case where the call is possible in the vehicle 11 and there is the connection request for implementing the call, the processing proceeds to step S125.

In step S125, the control unit 181 controls the communication unit 182 to set communication for implementing the call with the vehicle 11, and controls the voice call unit 184 to become able to accept an input of the voice uttered by the report recipient and output the voice from the vehicle 11 and the medical operator terminal 13.

By the processing, the report recipient who operates the report recipient terminal 12 becomes able to make a call with the driver or the passenger of the vehicle 11.

Similarly, in step S155, in the medical operator terminal 13, the control unit 191 controls the communication unit 192 to determine whether or not a call is possible in the vehicle 11 and there is a connection request for implementing the call. In step S155, in a case where the control unit 191 determines that the call is possible in the vehicle 11 and there is the connection request for implementing the call, the processing proceeds to step S156.

In step S156, the control unit 191 controls the communication unit 192 to set communication for implementing the call with the vehicle 11, and controls the voice call unit 194 to become able to accept an input of the voice uttered by the medical operator and output the voice from the vehicle 11 and the report recipient terminal 12. By the processing, the medical operator who operates the medical operator terminal 13 becomes able to make a call with the driver or the passenger of the vehicle 11 and the report recipient.

By the series of processing, the call among the driver or the passenger of the vehicle 11, the report recipient who operates the report recipient terminal 12, and the medical operator who operates the medical operator terminal 13 becomes possible.

Note that, in a case where the call is not possible in steps S93, S124, and S155, the processing in steps S194, S125, and S156 is skipped.

That is, in this case, the call among the driver or the passenger of the vehicle 11, the report recipient, and the medical operator cannot be made. However, even in the situation where the driver or the passenger cannot make a call, the report recipient and the medical operator may be able to make a call, or by making a call with the vehicle 11 possible, a passerby other than the driver or the passenger may get into the vehicle 11 and make a call at the site of the vehicle 11. That is, a call may be made possible regardless of whether or not the driver or the passenger can make a call.

In step S95, the abnormal situation processing unit 203 of the vehicle 11 determines whether or not the emergency call to the police station or medical institution is necessary from the vehicle interior information and the call content of the driver or the passenger supplied from the data acquisition unit 102.

In step S95, in a case where it is determined that the emergency call is necessary or the emergency call is requested from the driver or passenger, the processing proceeds to step S96.

In step S96, the abnormal situation processing unit 203 controls the communication unit 103 to transmit the emergency call to the report recipient terminal 12.

In response to the emergency call, in step S126, in the report recipient terminal 12, the control unit 181 controls the communication unit 182 to determine whether or not the emergency call has been transmitted from the vehicle 11. Then, in step S126, in a case where the emergency call has been transmitted, the processing proceeds to step S127.

In step S127, the control unit 181 controls the communication unit 182 to receive the emergency call transmitted from the vehicle 11 and transfer the emergency call to the medical operator terminal 13. At this time, the control unit 181 may control the communication unit 182 to send the emergency call to other facilities such as the police station and emergency medical facilities, if necessary.

Moreover, in step S157, in the medical operator terminal 13, the control unit 191 controls the communication unit 192 to determine whether or not the emergency call has been transmitted from the vehicle 11 via the report recipient terminal 12. Then, in step S157, in a case where the emergency call is transmitted, the processing proceeds to step S158.

In step S158, the control unit 191 controls the communication unit 192 to receive the emergency call transmitted from the vehicle 11 via the report recipient terminal 12. In this case, the control unit 191 also presents information indicating that the emergency call has been transmitted from the vehicle 11 to the display unit 193.

With the presentation, the medical operator who operates the medical operator terminal 13 can also recognize as information that the emergency call has been transmitted from the vehicle 11.

That is, by the series of processing, the medical operator judges the treatment for the driver in the abnormal physical condition on the basis of the vital data, the medical record information (in a case where the medical record has been searched for), and the presence or absence of the emergency call presented on the display unit 193 of the medical operator terminal 13, and operates a touch panel or the like of the display unit 193 and inputs the judgment result.

Here, the information to be input is the information of the necessity of the rescue by the emergency vehicle and the information of the medical institution required for treatment. By inputting only the necessary treatment, the medical institution capable of providing the treatment may be searched for from the position information of the vehicle 11.

Note that, in a case where it is determined that there is no emergency call in the processing in steps S95, S126, and S157, the processing in steps S96, S127, and S158 is skipped and it becomes a state where there is no emergency call.

Furthermore, the control unit 191 of the medical operator terminal 13 may have an agent function on behalf of the medical operator, and may determine the required medical treatment, specify the medical institution capable of providing the treatment, and determine the necessity of the rescue by the emergency vehicle from the information such as the medical record information, the vital data, and the presence or absence of the emergency call.

Note that the agent function that determines the medical treatment may be implemented by machine learning or the like using the information such as the medical record information, the vital data, and the presence or absence of the emergency call, and the corresponding treatment and the information of the presence or absence of the rescue by the emergency vehicle.

In step S159, the control unit 191 determines whether or not the rescue by the emergency vehicle is necessary according to whether or not the rescue by the emergency vehicle has been instructed by the operation of the medical operator on the touch panel of the display unit 193 or the determination result of the agent function.

In step S159, in a case where the rescue by the emergency vehicle is necessary, the processing proceeds to step S160.

In step S160, the control unit 191 controls the communication unit 192 to transmit the information requesting the rescue by the emergency vehicle and the information of the medical institution required for the treatment to the report recipient terminal 12.

Furthermore, in step S159, in a case where the rescue by the emergency vehicle is not necessary, the processing proceeds to step S161.

In step S161, the control unit 191 controls the communication unit 192 to transmit the information of the medical institution required for the treatment to the report recipient terminal 12.

In response to the transmission, in step S128, the control unit 181 of the report recipient terminal 12 controls the communication unit 192 to receive the information of the medical institution (including the information instructing the rescue by the emergency vehicle in a case where the rescue by the emergency vehicle is necessary) transmitted from the medical operator terminal 13.

In step S129, the control unit 181 determines whether or not the rescue by the emergency vehicle is necessary.

In step S129, in a case where the rescue by the emergency vehicle is necessary, the processing proceeds to step S130.

In step S130, the control unit 181 specifies the merging point between the vehicle 11 and the emergency vehicle in consideration of the transportation route to the medical institution from the position information of the medical institution based on the information of the specified medical institution and the position information of the vehicle 11.

In step S131, the control unit 181 controls the communication unit 182 to transmit the information of the specified medical institution and the position information of the merging point with the emergency vehicle to the vehicle 11. At this time, the control unit 181 controls the communication unit 182 to arrange the emergency vehicle to rescue the driver in the abnormal physical condition at the merging point and go to the specified medical institution.

On the other hand, in step S129, in a case where the rescue by the emergency vehicle is not necessary, the processing proceeds to step S132.

In step S132, the control unit 181 controls the communication unit 182 to transmit the information of the specified medical institution to the vehicle 11.

Then, in step S97, in the vehicle 11, the abnormal situation processing unit 203 controls the communication unit 103 to receive the information of the medical institution transmitted from the report recipient terminal 12, and determines whether or not the rescue by the emergency vehicle is necessary, that is, whether or not the position information of the merging point with the emergency vehicle is included.

In step S97, in a case where the position information of the merging point with the emergency vehicle is included and the rescue by the emergency vehicle is necessary, the processing proceeds to step S98.

In step S98, the abnormal situation processing unit 203 acquires the information of the medical institution for applying the medical treatment to the driver in the abnormal physical condition and the information of the merging point with the emergency vehicle.

In step S99, the abnormal situation processing unit 203 controls the information display unit 252 to present the information of the medical institution for applying the medical treatment to the driver in the abnormal physical condition, and move the vehicle 11 toward the merging point with the emergency vehicle.

More specifically, the abnormal situation processing unit 203 outputs the position information of the merging point with the emergency vehicle to the situation prediction unit 154.

The situation prediction unit 154 predicts moving the vehicle 11 to the position information of the merging point on the basis of the position information of the merging point with the emergency vehicle, and outputs a command instructing movement toward the merging point to the planning unit 134.

In the planning unit 134, the operation planning unit 163 plans an operation to move toward the merging point according to the command supplied from the situation prediction unit 154, and outputs the operation to the operation control unit 135.

In the operation control unit 135, the vehicle control unit 211 controls the drive system control unit 107 to operate the drive system 108 so as to move the vehicle 11 toward the merging point using the acceleration and deceleration control unit 172 and the direction control unit 173.

Furthermore, in step S97, in a case where the position information of the merging point with the emergency vehicle is not included and the rescue by the emergency vehicle is not necessary, the processing proceeds to step S100.

In step S100, the abnormal situation processing unit 203 acquires the information of the medical institution for applying the medical treatment to the driver in the abnormal physical condition.

In step S101, the abnormal situation processing unit 203 controls the information display unit 252 to present the information of the medical institution for applying the medical treatment to the driver in the abnormal physical condition, and move the vehicle 11 toward the medical institution.

By the above series of processing, in the case of the automatic driving level 5, the information indicating that the abnormal physical condition has occurred is reported together with the information that specifies the driver and the vital data even if the abnormal physical condition has occurred in the driver during driving. Furthermore, the information that specifies the driver and the vital data transmitted at the time of reporting are transmitted to the medical operator terminal 13 via the report recipient terminal 12, so the medical operator searches for the medical record according to the information that specifies the driver. Then, the medical operator judges the appropriate treatment for the driver in the abnormal physical condition and can specify the medical institution capable of providing the treatment on the basis of the vital data and the medical record information (in a case where the medical record has been searched for) .

Furthermore, at this time, if the driver or the passenger can make a call with the report recipient and the medical operator, the treatment to be applied to the driver in the abnormal physical condition can be appropriately judged on the basis of more detailed information by the call in addition to the vital data and the medical record information.

Moreover, the emergency call can be transmitted to the report recipient terminal 12 by the request from the driver or the passenger or by the determination of the abnormal situation processing unit 203.

Note that, in a case where the driver or the passenger cannot make a call or an emergency call, and the report recipient or the medical operator judges that the emergency call is necessary from the vital data, the image of the interior of the vehicle, or the like, the report recipient or the medical operator may issue the emergency call from the report recipient terminal 12 or the medical operator terminal 13.

Furthermore, in a case where the medical operator judges that the rescue by the emergency vehicle is necessary, the report recipient terminal 12 specifies the merging point between the emergency vehicle and the vehicle 11 in consideration of the transportation route to the medical institution, arranges the emergency vehicle to rush to the merging point, and transmits the position information of the merging point to the vehicle 11 to move to the merging point.

Thereby, since more prompt treatment can be applied to the driver in the abnormal physical condition, a lifesaving rate can be improved even in a case where the physical condition of the driver is serious.

Moreover, specification of the necessity of the emergency call, the required treatment content, and the medical institution capable of providing the treatment, determination of the necessity of the rescue by the emergency vehicle, and the like based on the vital data and the medical record information may be fully automated by causing the report recipient terminal 12 and the medical operator terminal 13 to have the agent function.

### <<11. Automatic Driving Level 4 Corresponding Processing>>

Next, the automatic driving level 4 corresponding processing will be described with reference to the flowchart in Fig. 13. Note that processing in steps S171 to S174 and S176 to S182, steps S201 to S209, S211, S212, and S214, and steps S221 to S231 in the flowchart in Fig. 13 is similar to the processing in the steps S91 to S101, steps S121 to S132, and steps S151 to S161 in Fig. 12, and thus the description thereof will be omitted.

That is, the difference in the flowchart in Fig. 13 from the flowchart in Fig. 12 is processing in steps S175, S183, and S184 and the processing in steps S210 and S213.

In step S175, the abnormal situation processing unit 203 determines whether or not replacement of the driver is necessary and the replacement of the driver is possible, or whether the replacement is not necessary.

In the case of the automatic driving level 4, the automatic driving is possible and the replacement of the driver is unnecessary when conditions for the automatic driving are satisfied, whereas the driver needs to be replaced to a driver capable of driving when the conditions for the automatic driving are not satisfied.

Therefore, in a case where the conditions for the automatic driving are not satisfied, the replacement is necessary, and thus whether or not the replacement of the driver is necessary and the replacement is possible, or whether the replacement is not necessary is determined.

Whether or not the replacement of the driver is necessary and the replacement is possible may be determined according to the presence or absence of declaration by the passenger about the replaceable driver, or when the passenger is not present or when the passenger is present but cannot respond due to occurrence of some failure, the abnormal situation processing unit 203 may determine that the replacement is not possible.

In step S175, in a case where the replacement of the driver is necessary and the replacement is possible, or the replacement of the driver is not necessary, the processing proceeds to step S176. Note that the processing in step S176 and subsequent steps is similar to the processing in step S95 and the subsequent steps in Fig. 12.

Furthermore, in step S176, in a case where the replacement of the driver is necessary but the replacement is not possible, it is determined that the automatic driving cannot be continued and the processing proceeds to step S183.

In step S183, the abnormal situation processing unit 203 stops the vehicle 11 at a safe place.

More specifically, the abnormal situation processing unit 203 notifies the situation prediction unit 154 of information indicating that the replacement of the driver is necessary but the replacement of the driver is not possible. In response to this notification, the situation prediction unit 154 predicts that the automatic driving cannot be continued, and notifies the planning unit 134 of a command instructing stop of the vehicle 11 at a safe place.

In the planning unit 134, the operation planning unit 163 plans an operation to stop at a safe place according to the command supplied from the situation prediction unit 154, and outputs the operation to the operation control unit 135.

In the operation control unit 135, the vehicle control unit 211 controls the drive system control unit 107 to operate the drive system 108 so as to stop the vehicle 11 at a safe place using the acceleration and deceleration control unit 172 and the direction control unit 173.

In step S184, the abnormal situation processing unit 203 controls the communication unit 103 to transmit a notification requesting the rescue by the emergency vehicle to the site to the report recipient terminal 12.

By the series of processing, the vehicle 11 waits for the rescue by the emergency vehicle while stopped at a safe position.

Furthermore, in step S210, in the report recipient terminal 12, the control unit 181 controls the communication unit 182 to determine whether or not the rescue by the emergency vehicle to the site is requested. That is, whether or not the rescue by the emergency vehicle is requested and the rescue to the site at the position where the vehicle 11 is stopped is requested is determined.

In step S210, in a case where the rescue by the emergency vehicle is requested, the processing proceeds to step S213.

In step S213, the control unit 181 controls the communication unit 182 to transmit the position information of the vehicle 11 and arrange the emergency vehicle to go to the site where the vehicle 11 is stopped.

By the above processing, the emergency vehicle goes to rescue the driver in the abnormal physical condition at the position of the vehicle 11 stopped at a safe place.

Thereby, when the automatic driving level is the level 4 and even if the replacement of the driver is necessary but the replacement of the driver is not possible, the vehicle 11 is stopped at a safe place and the driver in the abnormal physical condition is rescued by the emergency vehicle and is then transported to the medical institution capable of providing the necessary treatment.

Note that, at the automatic driving level 4, in a case where the replacement of the driver is necessary and the replacement of the driver is possible, the automatic driving cannot be continued. Therefore, in steps S180 and S182, the replaced driver needs to drive the vehicle 11 and move to the medical institution or the merging point with the emergency vehicle.

Therefore, in step S180, the abnormal situation processing unit 203 controls the information display unit 252 to present the information of the medical institution for applying the medical treatment to the driver in the abnormal physical condition and the merging point with the emergency vehicle, and present information for urging the replaced driver to drive and move the vehicle 11 toward the merging point with the emergency vehicle. With this presentation, the replaced driver drives the vehicle 11 and moves toward the merging point with the emergency vehicle. Then, at the merging point, the driver in the abnormal physical condition is rescued by the emergency vehicle and transported to the medical institution, and the necessary treatment is applied to the driver in the abnormal physical condition.

Furthermore, in step S182, the abnormal situation processing unit 203 controls the information display unit 252 to present the information of the medical institution for applying the medical treatment to the driver in the abnormal physical condition, and present the information for urging the replaced driver to drive and move the vehicle 11 toward the medical institution. By the presentation, the replaced driver drives and moves the vehicle 11 toward the medical institution. Then, in the medical institution, the necessary treatment is applied to the driver in the abnormal physical condition.

As a result, in a case where the automatic driving level is the level 4 and even in the state where the replacement of the driver is necessary and the replacement is not possible, the driving control appropriately considering safety for the driver in the abnormal physical condition and the passenger, and the surrounding vehicles and passersby can be implemented.

### <<12. Automatic Driving Level 3 Corresponding Processing>>

Next, the automatic driving level 3 corresponding processing will be described with reference to the flowchart in Fig. 14. Note that processing in steps S252 to S264, steps S281 to S294, and steps S311 to S321 in the flowchart in Fig. 14 is similar to the processing in the steps S171 to S182 and S184, steps S201 to S214, and steps S221 to S231 in Fig. 13, and thus the description thereof will be omitted.

That is, the difference in the flowchart in Fig. 14 from the flowchart in Fig. 13 is that, in step S251, the abnormal situation processing unit 203 stops the vehicle 11 at a safe place before the processing in step S252 for notifying that the abnormal physical condition has occurred in the driver.

That is, in a case where the automatic driving level is the level 3, the state where the driver can drive is the condition for the automatic driving. Therefore, the automatic driving becomes not possible at the time when the abnormal physical condition has occurred in the driver, and thus the vehicle 11 is stopped at a safe place in the initial processing.

The subsequent processing is similar to the processing in a case where the automatic driving level is the level 4 described with reference to the flowchart in Fig. 13.

By the above processing, even when the automatic driving level is the level 3, the vehicle 11 is stopped at a safe place and the driver in the abnormal physical condition is transported to the medical institution capable of providing the necessary treatment.

As a result, even if the automatic driving level is the level 3, processing appropriately considering safety can be performed.

### <<13. Emergency Stop Support Processing>>

Next, the emergency stop support processing will be described with reference to the flowchart in Fig. 15. Note that processing in steps S332 to S344, steps S361 to S374, and steps S391 to S401 in the flowchart in Fig. 15 are similar to the processing in steps S252 to S264, steps S281 to S294, and steps S311 to S321 in Fig. 14, and thus the description thereof will be omitted.

That is, the difference in the flowchart in Fig. 15 from the flowchart in Fig. 14 in that the abnormal situation processing unit 203 decelerates and stops the vehicle 11 while turning on the hazard lights in step S331.

That is, in a case where the automatic driving level is one of the levels 0 to 2, when the driver is in the abnormal physical condition, the driver becomes unable to drive at the time when the abnormal physical condition has occurred in the driver, and moreover a safe place cannot be selected. Therefore, the vehicle 11 is decelerated and stopped even on a driving lane while the hazard lights are turned on.

The subsequent processing is similar to the processing in a case where the automatic driving level is the level 4 described with reference to the flowchart in Fig. 13.

Note that, in a case where the automatic driving level is one of the levels 0 to 2, replacement of the driver is required as soon as the abnormal physical condition has occurred in the driver. Therefore, in step S336, whether or not the replacement of the driver is possible is determined instead of whether or not the replacement of the driver is necessary and the replacement is possible, or whether or not the replacement of the driver is unnecessary. Then, in step S336, when the replacement of the driver is possible, the processing proceeds to step S337, and the replaced driver drives the vehicle 11 and moves the vehicle 11 to the merging point or the medical institution. Furthermore, in step S336, when the replacement of the driver is not possible, the processing proceeds to step S344, and the emergency vehicle is arranged to the site where the vehicle 11 is stopped.

By the above processing, even when the automatic driving level is one of the levels 0 to 2, the vehicle 11 is decelerated and stopped on the driving lane with the hazard lights on, and the driver in the abnormal physical condition is transported by the vehicle 11 by the replaced driver to the medical institution or the merging point or by the emergency vehicle to the medical institution.

As a result, even in a case where the automatic driving level is one of the levels 0 to 2, the driving control appropriately considering safety for the driver in the abnormal physical condition and the passenger, and the surrounding vehicles and passersby can be implemented.

### <<14. Example of Execution by Software>>

By the way, the above-described series of processing can be executed by hardware or software. In a case where the series of processing is executed by software, a program constituting the software is installed from a recording medium into a computer incorporated in special hardware, a general-purpose computer capable of executing various functions by installing various programs, or the like.

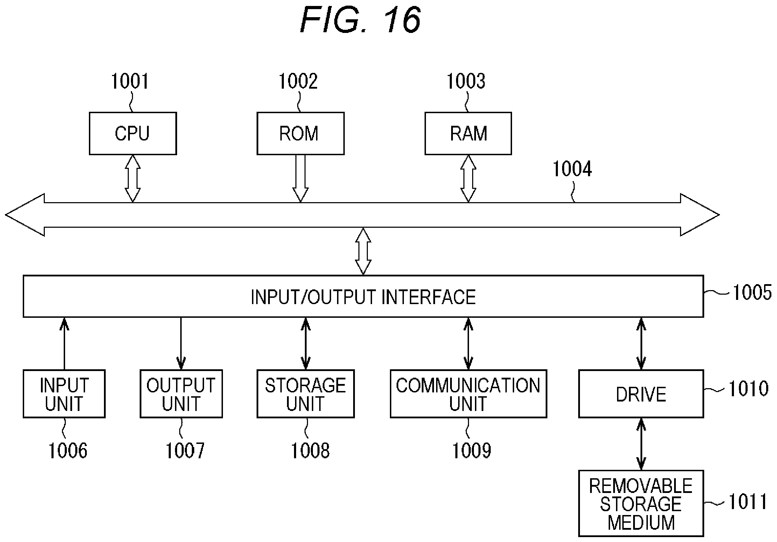

Fig. 16 illustrates a configuration example of a general-purpose computer. The computer incorporates a central processing unit (CPU) 1001. An input/output interface 1005 is connected to the CPU 1001 via a bus 1004. A read only memory (ROM) 1002 and a random access memory (RAM) 1003 are connected to the bus 1004.

To the input/output interface 1005, an input unit 1006 including an input device such as a keyboard and a mouse for a user to input operation commands, an output unit 1007 that outputs a processing operation screen and an image of a processing result to a display device, a storage unit 1008 including a hard disk drive for storing programs and various data, and a communication unit 1009 including a local area network (LAN) adapter and the like and which executes communication processing via a network typified by the Internet are connected. Furthermore, a drive 1010 that reads and writes data with respect to a removable recording medium 1011 such as a magnetic disk (including a flexible disk), an optical disk (including a compact disc-read only memory (CD-ROM) or a digital versatile disc (DVD)), a magneto-optical disk (including a mini disc (MD)), or a semiconductor memory is connected to the input/output interface 1005.

The CPU 1001 executes various types of processing according to a program stored in the ROM 1002 or a program read from the removable recording medium 1011 such as a magnetic disk, an optical disk, a magneto-optical disk, or a semiconductor memory, installed in the storage unit 1008, and loaded from the storage unit 1008 to the RAM 1003. Furthermore, the RAM 1003 appropriately stores data and the like necessary for the CPU 1001 to execute the various types of processing.

In the computer configured as described above, the CPU 1001, for example, loads the program stored in the storage unit 1008 into the RAM 1003 and executes the program via the input/output interface 1005 and the bus 1004, whereby the above-described series of processing is performed.

The program to be executed by the computer (CPU 1001) can be recorded on the removable recording medium 1011 as a package medium or the like, for example, and provided. Furthermore, the program can be provided via a wired or wireless transmission medium such as a local area network, the Internet, or digital satellite broadcast.

In the computer, the program can be installed to the storage unit 1008 via the input/output interface 1005 by attaching the removable recording medium 1011 to the drive 1010. Furthermore, the program can be received by the communication unit 1009 via a wired or wireless transmission medium and installed in the storage unit 1008. Other than the above method, the program can be installed in the ROM 1002 or the storage unit 1008 in advance.

Note that the program executed by the computer may be a program processed in chronological order according to the order described in the present specification or may be a program executed in parallel or at necessary timing such as when a call is made.

Note that the CPU 1001 in Fig. 16 implements the function of the automatic driving control unit 112 in Fig. 8. Furthermore, the storage unit 1008 in Fig. 16 implements the storage unit 111 in Fig. 8.

Furthermore, in the present specification, the term "system" means a group of a plurality of configuration elements (devices, modules (parts), and the like), and whether or not all the configuration elements are in the same casing is irrelevant. Therefore, a plurality of devices housed in separate casings and connected via a network, and one device that houses a plurality of modules in one casing are both systems.

Note that the embodiments of the present disclosure are not limited to the above-described embodiments, and various modifications can be made without departing from the gist of the present disclosure.

For example, the present disclosure can adopt a configuration of cloud computing in which one function is shared and processed in cooperation by a plurality of devices via a network.

Furthermore, the steps described in the above-described flowcharts can be executed by one device or can be shared and executed by a plurality of devices.

Moreover, in a case where a plurality of processes is included in one step, the plurality of processes included in the one step can be executed by one device or can be shared and executed by a plurality of devices.

Note that the present disclosure can have the following configurations.

<1> An information processing apparatus including:
   a vital data acquisition unit configured to acquire vital data of a driver of a vehicle; and
   an abnormal situation processing unit configured to control the vehicle to a safe state according to an automatic driving level in a case of acquiring a determination result that an abnormal physical condition is occurring in the driver on the basis of the vital data.
<2> The information processing apparatus according to <1>, further including:
   an abnormal physical condition determination unit configured to determine whether or not the abnormal physical condition is occurring in the driver on the basis of the vital data.
<3> The information processing apparatus according to <2>, in which
   the abnormal physical condition determination unit determines whether or not the abnormal physical condition is occurring in the driver on the basis of the vital data by machine learning.
<4> The information processing apparatus according to claim 2, further including:
   a communication unit configured to transmit the vital data; and
   an acquisition unit configured to acquire the determination result that the abnormal physical condition is occurring in the driver on the basis of the vital data, in which
   the abnormal physical condition determination unit determines whether or not the abnormal physical condition is occurring in the driver according to the acquired determination result.
<5> The information processing apparatus according to <4>, in which
   the communication unit transmits the vital data to a specialized medical institution that determines presence or absence of occurrence of the abnormal physical condition on the basis of the vital data,
   the acquisition unit acquires a determination result of the presence or absence of occurrence of the abnormal physical condition based on the vital data by the specialized medical institution, and
   the abnormal physical condition determination unit determines whether or not the abnormal physical condition is occurring in the driver according to the determination result of the presence or absence of occurrence of the abnormal physical condition based on the vital data by the specialized medical institution.
<6> The information processing apparatus according to <2>, in which
   the abnormal physical condition determination unit determines whether or not the abnormal physical condition is occurring in the driver on the basis of the vital data in a case where the automatic driving level is level 4 or level 5.
<7> The information processing apparatus according to <2>, in which,
   in a case where the automatic driving level is level 4 or level 5, when it is determined that the abnormal physical condition is occurring in the driver on the basis of the vital data, the abnormal situation processing unit transmits a notification indicating that the abnormal physical condition is occurring in the driver together with the vital data and receives information of a medical institution capable of providing treatment to be applied to the driver in the abnormal physical condition based on the vital data, the information of a medical institution being transmitted in response to the notification, and controls the vehicle to the safe state by operating the vehicle to move to the medical institution.
<8> The information processing apparatus according to <7>, in which
   the abnormal situation processing unit receives the information of a medical institution capable of providing treatment to be applied to the driver in the abnormal physical condition based on the vital data, the information being transmitted in response to the notification, and information indicating necessity of a rescue by an emergency vehicle, and controls the vehicle to the safe state by operating the vehicle to move to a merging point with the emergency vehicle when the rescue by the emergency vehicle is necessary.
<9> The information processing apparatus according to <7>, in which
   the abnormal situation processing unit transmits the notification indicating that the abnormal physical condition is occurring in the driver together with the vital data and information that specifies the driver and receives the information of a medical institution capable of providing treatment to be applied to the driver in the abnormal physical condition based on the vital data and information of a medical record searched with the information that specifies the driver, the information of a medical institution being transmitted in response to the notification, and controls the vehicle to the safe state by operating the vehicle to move to the medical institution.
<10> The information processing apparatus according to <7>, in which,
   in a case where the automatic driving level is level 4, when it is determined that the abnormal physical condition is occurring in the driver on the basis of the vital data, and in a case where replacement of the driver is necessary and the replacement is possible or in a case where the replacement of the driver is not necessary, the abnormal situation processing unit controls the vehicle to the safe state by operating the vehicle to move to the medical institution.
<11> The information processing apparatus according to <7>, in which,
   in a case where the automatic driving level is level 4, when it is determined that the abnormal physical condition is occurring in the driver on the basis of the vital data, and replacement of the driver is necessary but the replacement is not possible, the abnormal situation processing unit controls the vehicle to the safe state by operating the vehicle to stop at a safe place, or operating the vehicle to stop at the safe place and requesting a rescue by an emergency vehicle to a position where the vehicle has stopped.
<12> The information processing apparatus according to <2>, in which,
   in a case where the automatic driving level is level 3, when it is determined that the abnormal physical condition is occurring in the driver on the basis of the vital data, the abnormal situation processing unit transmits a notification indicating that the abnormal physical condition is occurring in the driver together with the vital data and receives information of a medical institution capable of providing treatment to be applied to the driver in the abnormal physical condition based on the vital data, the information of a medical institution being transmitted in response to the notification, after operating the vehicle to stop at the safe place, and controls the vehicle to the safe state by presenting information that urges the vehicle to move to the medical institution.
<13> The information processing apparatus according to <12>, in which,
   in a case where the automatic driving level is level 3, when it is determined that the abnormal physical condition is occurring in the driver on the basis of the vital data, and in a case where replacement of the driver is necessary but the replacement is not possible, the abnormal situation processing unit controls the vehicle to the safe state by requesting a rescue by an emergency vehicle to a position where the vehicle has stopped.
<14> The information processing apparatus according to <2>, in which,
   in a case where the automatic driving level is one of levels 0 to 2, when it is determined that the abnormal physical condition is occurring in the driver on the basis of the vital data, the abnormal situation processing unit transmits a notification indicating that the abnormal physical condition is occurring in the driver together with the vital data and receives information of a medical institution capable of providing treatment to be applied to the driver in the abnormal physical condition based on the vital data, the information of a medical institution being transmitted in response to the notification, after operating the vehicle to turn on hazard lights and stop while decelerating, and controls the vehicle to the safe state by presenting information that urges the vehicle to move to the medical institution.
<15> The information processing apparatus according to <14>, in which,
   in a case where the automatic driving level is one of levels 0 to 2, when it is determined that the abnormal physical condition is occurring in the driver on the basis of the vital data, and in a case where replacement of the driver is not possible, the abnormal situation processing unit controls the vehicle to the safe state by requesting a rescue by an emergency vehicle to a position where the vehicle has stopped.
<16> The information processing apparatus according to any one of <2> to <5>, in which,
   in a case where the automatic driving level is one of levels 0 to 3, and when a line-of-sight of the driver is not able to be detected, the abnormal physical condition determination unit determines whether or not the abnormal physical condition is occurring in the driver on the basis of the vital data.
<17> The information processing apparatus according to <16>, in which,
   in a case where the line-of-sight of the driver is not able to be detected, and when a feedback that encourages arousal is given to the driver and the line-of-sight is still not able to be detected, the abnormal physical condition determination unit determines whether or not the abnormal physical condition is occurring in the driver on the basis of the vital data.
<18> The information processing apparatus according to <17>, in which,
   in a case where the line-of-sight of the driver is not able to be detected, and when the feedback that encourages arousal is given to the driver and a state in which the line-of-sight cannot be detected continues, the abnormal physical condition determination unit repeats the feedback and determines whether or not the abnormal physical condition is occurring in the driver according to whether or not the feedback has been repeated a predetermined number of times.
<19> An information processing method including:
   vital data acquisition processing of acquiring vital data of a driver of a vehicle; and
   abnormal situation processing of controlling the vehicle to a safe state according to an automatic driving level in a case of acquiring a determination result that an abnormal physical condition is occurring in the driver on the basis of the vital data.
<20> A program for causing a computer to function as:
   a vital data acquisition unit configured to acquire vital data of a driver of a vehicle; and
   an abnormal situation processing unit configured to control the vehicle to a safe state according to an automatic driving level in a case of acquiring a determination result that an abnormal physical condition is occurring in the driver on the basis of the vital data.

### REFERENCE SIGNS LIST

- 11: Vehicle
- 12: Report recipient terminal
- 13: Medical operator terminal
- 100: Vehicle control system
- 102: Data acquisition unit
- 112: Automatic driving control unit
- 133: Situation analysis unit
- 134: Planning unit
- 135: Operation control unit
- 153: Situation recognition unit
- 154: Situation prediction unit
- 172: Acceleration and deceleration control unit
- 173: Direction control unit
- 185: Vital data transmission/reception management unit
- 195: Medical record management unit
- 201: Vehicle interior environment recognition unit
- 202: Feedback control unit
- 203: Abnormal situation processing unit
- 204: E-call
- 211: Vehicle control unit
- 231: In-vehicle sensor
- 232: Vital data acquisition sensor
- 251: Irritating odor generation unit
- 252: Information display unit
- 253: Speaker

## Claims

1. An information processing apparatus comprising:
a vital data acquisition unit configured to acquire vital data of a driver of a vehicle; and
an abnormal situation processing unit configured to control the vehicle to a safe state according to an automatic driving level in a case of acquiring a determination result that an abnormal physical condition is occurring in the driver on a basis of the vital data.

2. The information processing apparatus according to claim 1, further comprising:
an abnormal physical condition determination unit configured to determine whether or not the abnormal physical condition is occurring in the driver on a basis of the vital data.

3. The information processing apparatus according to claim 2, wherein
the abnormal physical condition determination unit determines whether or not the abnormal physical condition is occurring in the driver on a basis of the vital data by machine learning.

4. The information processing apparatus according to claim 2, further comprising:
a communication unit configured to transmit the vital data; and
an acquisition unit configured to acquire the determination result that the abnormal physical condition is occurring in the driver on a basis of the vital data, wherein
the abnormal physical condition determination unit determines whether or not the abnormal physical condition is occurring in the driver according to the acquired determination result.

5. The information processing apparatus according to claim 4, wherein
the communication unit transmits the vital data to a specialized medical institution that determines presence or absence of occurrence of the abnormal physical condition on a basis of the vital data,
the acquisition unit acquires a determination result of the presence or absence of occurrence of the abnormal physical condition based on the vital data by the specialized medical institution, and
the abnormal physical condition determination unit determines whether or not the abnormal physical condition is occurring in the driver according to the determination result of the presence or absence of occurrence of the abnormal physical condition based on the vital data by the specialized medical institution.

6. The information processing apparatus according to claim 2, wherein
the abnormal physical condition determination unit determines whether or not the abnormal physical condition is occurring in the driver on a basis of the vital data in a case where the automatic driving level is level 4 or level 5.

7. The information processing apparatus according to claim 2, wherein,
in a case where the automatic driving level is level 4 or level 5, when it is determined that the abnormal physical condition is occurring in the driver on a basis of the vital data, the abnormal situation processing unit transmits a notification indicating that the abnormal physical condition is occurring in the driver together with the vital data and receives information of a medical institution capable of providing treatment to be applied to the driver in the abnormal physical condition based on the vital data, the information of a medical institution being transmitted in response to the notification, and controls the vehicle to the safe state by operating the vehicle to move to the medical institution.

8. The information processing apparatus according to claim 7, wherein
the abnormal situation processing unit receives the information of a medical institution capable of providing treatment to be applied to the driver in the abnormal physical condition based on the vital data, the information being transmitted in response to the notification, and information indicating necessity of a rescue by an emergency vehicle, and controls the vehicle to the safe state by operating the vehicle to move to a merging point with the emergency vehicle when the rescue by the emergency vehicle is necessary.

9. The information processing apparatus according to claim 7, wherein
the abnormal situation processing unit transmits the notification indicating that the abnormal physical condition is occurring in the driver together with the vital data and information that specifies the driver and receives the information of a medical institution capable of providing treatment to be applied to the driver in the abnormal physical condition based on the vital data and information of a medical record searched with the information that specifies the driver, the information of a medical institution being transmitted in response to the notification, and controls the vehicle to the safe state by operating the vehicle to move to the medical institution.

10. The information processing apparatus according to claim 7, wherein,
in a case where the automatic driving level is level 4, when it is determined that the abnormal physical condition is occurring in the driver on a basis of the vital data, and in a case where replacement of the driver is necessary and the replacement is possible or in a case where the replacement of the driver is not necessary, the abnormal situation processing unit controls the vehicle to the safe state by operating the vehicle to move to the medical institution.

11. The information processing apparatus according to claim 7, wherein,
in a case where the automatic driving level is level 4, when it is determined that the abnormal physical condition is occurring in the driver on a basis of the vital data, and replacement of the driver is necessary but the replacement is not possible, the abnormal situation processing unit controls the vehicle to the safe state by operating the vehicle to stop at a safe place, or operating the vehicle to stop at the safe place and requesting a rescue by an emergency vehicle to a position where the vehicle has stopped.

12. The information processing apparatus according to claim 2, wherein,
in a case where the automatic driving level is level 3, when it is determined that the abnormal physical condition is occurring in the driver on a basis of the vital data, the abnormal situation processing unit transmits a notification indicating that the abnormal physical condition is occurring in the driver together with the vital data and receives information of a medical institution capable of providing treatment to be applied to the driver in the abnormal physical condition based on the vital data, the information of a medical institution being transmitted in response to the notification, after operating the vehicle to stop at the safe place, and controls the vehicle to the safe state by presenting information that urges the vehicle to move to the medical institution.

13. The information processing apparatus according to claim 12, wherein,
in a case where the automatic driving level is level 3, when it is determined that the abnormal physical condition is occurring in the driver on a basis of the vital data, and in a case where replacement of the driver is necessary but the replacement is not possible, the abnormal situation processing unit controls the vehicle to the safe state by requesting a rescue by an emergency vehicle to a position where the vehicle has stopped.

14. The information processing apparatus according to claim 2, wherein,
in a case where the automatic driving level is one of levels 0 to 2, when it is determined that the abnormal physical condition is occurring in the driver on a basis of the vital data, the abnormal situation processing unit transmits a notification indicating that the abnormal physical condition is occurring in the driver together with the vital data and receives information of a medical institution capable of providing treatment to be applied to the driver in the abnormal physical condition based on the vital data, the information of a medical institution being transmitted in response to the notification, after operating the vehicle to turn on hazard lights and stop while decelerating, and controls the vehicle to the safe state by presenting information that urges the vehicle to move to the medical institution.

15. The information processing apparatus according to claim 14, wherein,
in a case where the automatic driving level is one of levels 0 to 2, when it is determined that the abnormal physical condition is occurring in the driver on a basis of the vital data, and in a case where replacement of the driver is not possible, the abnormal situation processing unit controls the vehicle to the safe state by requesting a rescue by an emergency vehicle to a position where the vehicle has stopped.

16. The information processing apparatus according to claim 2, wherein,
in a case where the automatic driving level is one of levels 0 to 3, and when a line-of-sight of the driver is not able to be detected, the abnormal physical condition determination unit determines whether or not the abnormal physical condition is occurring in the driver on a basis of the vital data.

17. The information processing apparatus according to claim 16, wherein,
in a case where the line-of-sight of the driver is not able to be detected, and when a feedback that encourages arousal is given to the driver and the line-of-sight is still not able to be detected, the abnormal physical condition determination unit determines whether or not the abnormal physical condition is occurring in the driver on a basis of the vital data.

18. The information processing apparatus according to claim 17, wherein,
in a case where the line-of-sight of the driver is not able to be detected, and when the feedback that encourages arousal is given to the driver and a state in which the line-of-sight cannot be detected continues, the abnormal physical condition determination unit repeats the feedback and determines whether or not the abnormal physical condition is occurring in the driver according to whether or not the feedback has been repeated a predetermined number of times.

19. An information processing method comprising:
vital data acquisition processing of acquiring vital data of a driver of a vehicle; and
abnormal situation processing of controlling the vehicle to a safe state according to an automatic driving level in a case of acquiring a determination result that an abnormal physical condition is occurring in the driver on a basis of the vital data.

20. A program for causing a computer to function as:
a vital data acquisition unit configured to acquire vital data of a driver of a vehicle; and
an abnormal situation processing unit configured to control the vehicle to a safe state according to an automatic driving level in a case of acquiring a determination result that an abnormal physical condition is occurring in the driver on a basis of the vital data.
